# EUROPEAN PATENT SPECIFICATION

(11) **EP 4 034 634 B1**
(45) Date of publication and mention of the grant of the patent: **01.01.2025**
(21) Application number: 20775022.5
(22) Date of filing: 24.09.2020
(51) Int. Cl.: C12N 5/00, C07K 14/47, C08C 19/00

(54) **CELL CULTURING MATERIALS**
ZELLKULTURMATERIALEN
MATÉRIAUX DE CULTURE DE CELLULES

(30) Priority: 24.09.2019 EP 19199362
(43) Date of publication of application: 03.08.2022
(60) Divisional application: 24202851.2
(73) Proprietor: Koninklijke Philips N.V., 5656 AG Eindhoven (NL)
(72) Inventor: BECKERS, Lucas, Johannes, Anna, Maria, 5656 AE Eindhoven (NL); VAN GIJSEL, Thomas, Johannes, 5656 AE Eindhoven (NL)
(74) Representative: Philips Intellectual Property & Standards
(86) International application number: PCT/EP2020/076749
(87) International publication number: WO 2021/058657

(56) References cited:
- WO-A1-2019/015988
- WO-A2-2008/150947
- US-A1- 2009 186 411
- US-A1- 2011 275 539
- D. BIELINSKI ET AL: "Surface migration of carboxylic acid in styrene-butadiene rubber and its tribological consequences", JOURNAL OF APPLIED POLYMER SCIENCE, vol. 86, no. 13, 9 October 2002 (2002-10-09), US, pages 3368 - 3376, XP055499162, ISSN: 0021-8995, DOI: 10.1002/app.11321
- S. PINTO ET AL: "Poly(dimethyl siloxane) surface modification with biosurfactants isolated from probiotic strains", JOURNAL OF BIOMEDICAL MATERIALS RESEARCH PART A, vol. 98A, no. 4, 15 September 2011 (2011-09-15), pages 535 - 543, XP055429681, ISSN: 1549-3296, DOI: 10.1002/jbm.a.33146
- MARY L. KRAFT ET AL: "Multitechnique Characterization of Fatty Acid-Modified Microgels", LANGMUIR, vol. 20, 22 January 2004 (2004-01-22), pages 1111 - 1119, XP055428560, DOI: 10.1021/la035617j
- JAN SCHWARZ ET AL: "A microfluidic device for measuring cell migration towards substrate-bound and soluble chemokine gradients", SCIENTIFIC REPORTS, vol. 6, no. 1, 7 November 2016 (2016-11-07), XP055428717, DOI: 10.1038/srep36440

## Description

### FIELD OF THE INVENTION

The invention relates to a cell culturing substrate comprising an elastomer material for culturing cells.

The invention further relates to a fluidic device module and fluidic device comprising such a cell culturing substrate.

The invention further relates to a method of culturing cells.

The invention further relates to a drug testing method with a fluidic device module comprising such an elastomeric material.

The invention relates to use of an elastomer material for cell culturing.

The invention relates to manufacture of a cell culturing substrate.

### BACKGROUND OF THE INVENTION

In-vitro testing of mammalian cells or tissue is an important technique to obtain clinically important information of the mammalian material under investigation. For example, biopsied mammalian cell or tissue material may be subjected to such testing in order to determine anomalies or disease in such mammalian material or to expose diseased mammalian material to drugs, e.g. experimental drugs, to monitor the response of the diseased mammalian material to such exposure. This approach for example is frequently used in oncological procedures. This can provide important insights in how a disease in an individual can be effectively treated without having to expose the individual to a range of potentially effective drugs, which can be undesirable for a number of reasons, such as drug toxicity. In addition, the efficacy of experimental drugs for existing diseases for which no established satisfactory drug treatment is yet available may be tested in this manner. There are many other well-known reasons for deploying such in-vitro tests.

A common approach to such in-vitro testing is to immobilize the mammalian material in a fluidic device, which is sometimes referred to as an organ-on-chip. In such an approach, the mammalian material is typically immobilized on a membrane separating two fluidic channels of the fluidic device, with a first channel being used to feed the mammalian material and the second channel being used to expose the mammalian material to a chemical compound or composition of interest such as a drug treatment, e.g. to test the efficacy and/or toxicity of the drug as previously explained. The fluidic device, or at least its membrane, may be made of an elastomer such that the fluidic device may be diced or sliced in order to obtain a slice of the membrane including the mammalian material for evaluation purposes, e.g. to evaluate the reaction of the mammalian material to the exposure to the chemical compound or composition of interest.

A challenge in such in-vitro testing is to ensure that the mammalian material, e.g. cells or tissue, are stabilized and develop normally on the membrane of the fluidic device such that they live for the duration of the test procedure. This is particularly challenging in oncology procedures where experiments can be rather lengthy. To this end, the mammalian material may be stabilized using a biocompatible material such as fibronectin, which ensures that the mammalian cells will keep multiplying by providing the cells with a chemical environment mimicking that of natural tissue.

A common approach is to coat a well cell plate with a polymer having carboxylic acid groups, as such groups can (covalently) bond to the fibronectin, thereby facilitating the stabilization of the mammalian cells on the membrane within the fluidic device as the fibronectin makes the polymer bioconnective. However, this approach is not without its disadvantages. Firstly, the fluidic streams through the fluidic device may partially remove the aforementioned polymer, which compromises the ability to evaluate the mammalian material at a desired point in time due to the fact that at least some of this material may have been lost. In another approach, the membrane is treated with UV or plasma to generate binding sites for the fibronectin on the membrane. However, this approach has the drawback that it is rather difficult to avoid an inhomogeneous distribution of such binding sites, which again hampers evaluation of testing results.

Secondly, membranes comprising such a biocompatible coating are notoriously difficult to manufacture, which makes the manufacture of such fluidic devices rather costly and cumbersome. For example, such membranes typically need to be manufactured using thin-film technology, which is expensive, and need to be integrated within the fluidic device without leakage, which is far from straightforward. In addition, where the membrane itself is not made of a biocompatible material the membrane typically needs to be provided with a plurality of cell-sized holes with a small pitch to facilitate cell growth, which is also difficult to achieve.

An example of the microfabrication of such a human organ-on-chip based on thin film techniques is disclosed by Dongeun Huh et al. in Nature Protocols, Vol. 8, No. 11, 2013, pages 2135-2157. In this protocol, micro-engineering is used to fabricate a multilayered microfluidic device containing two parallel elastomeric micro-channels separated by a thin porous flexible membrane along with two full-height, although vacuum chambers on either side, which device takes approximately 3 ½ days to produce. The total microfabrication procedure includes over 100 steps, with a significant number of them being critical steps. This clearly demonstrates the complexity of such manufacturing procedures.

An improvement over such laborious approaches is disclosed in WO 2018/021906 A1, which discloses a silicon-based (PDMS) fluidic device that can be made biocompatible using a coating of e.g. collagen. Such a device may be manufactured in a few manufacturing steps only, but still suffers from the drawback that it needs coating with a biocompatible material, which as explained above can erode from the polymer surface when exposed to fluid streams, thus leading to the unwanted loss of cell material from the fluidic device surface. This is addressed in WO 2018/021906 A1 by plasma-treating the surface of the device and subsequently functionalizing the surface with 3-aminopropyltriethoxysilane and glutaraldehyde prior to coating the functionalized surface with collagen and removing unbound collagen by flushing the microchannels of the fluidic device with EGM-2 (Endothelial Cell Growth Medium-2 BulletKit as marketed by the Lonza corporation). Hence, a substantial number of processing steps are still required to provide a biocompatible fluidic device.

A solution to this problem is disclosed by the applicant in WO 2019/015988 A1. In this application, a material for binding to a cell culturing protein is disclosed. The material contains a bulk-modified elastomer comprising a plurality of fatty acid moieties covalently bound to the elastomer bulk, wherein the carboxylic acid groups of said moieties are available to provide said binding on the surface of an object formed from the bulk-modified elastomer by bulk-modifying the elastomer with the fatty acid moieties in a mold in the shape of the object and having a polar inner surface such that the hydrophilic carboxylic acid groups of the fatty acid moieties are attracted to this polar inner surface. It has been demonstrated in this prior application that a biocompatible material such as fibronectin may be covalently bound to the surface carboxylic acid groups of the bulk-modified elastomer whilst retaining its cell culturing properties.

There is a need for further improvement in cell culturing substrates and their manufacture.

### SUMMARY OF THE INVENTION

This need is at least partly fulfilled by the current invention which seeks to provide several aspects as defined by the independent claims each of which either makes use of or provides an elastomeric material for culturing cells without having to covalently modify the surface with a cell culturing protein.

According to an aspect, there is provided a cell culturing substrate according to claim 1.

The term substrate adapted to culture cells or cell culturing substrate is meant to include any device or surface that is intended for use in cell culturing. It is intended that the substrate defined in this way embodies the use of the elastomeric material as defined herein in the field of cell culturing. Cell culturing is meant to include e.g. keeping cells alive and/or multiply the number of cells and/or differentiate cells. A residue is a part of a precursor molecule carrying the acidic group in either of the forms and an ethylenic bond capable of participating in a reaction to covalently bond the precursor to the elastomer bulk. The residue is the part of the precursor that remains after such reaction.

It has been found that when such surface is directly brought in contact with a cell culturing medium in the presence of cells, such cells can be cultivated without any further surface preparation. Hence the step of covalently modifying the material surface with a cell culturing protein can be omitted thus facilitating use. While not wanting to be bound by theory, it is believed that the acidic groups available at the surface are playing an important role for this cell culturing function. After all, reference non bulk-modified elastomers did not show such cell culturing properties. It was also found that the acidic groups may be present at the surface in either free form meaning that they carry a proton capable of dissociating or in conjugated base form accompanied by a positive counter ion other than a proton. Without wanting to be bound by theory, it is believed that in the cell culturing medium, which is typically buffered at pH around 7.2, such acidic groups with pKa values typically less than 5.5 are present in dissociated form to a large extent regardless of their original form in which they are available at the surface.

The acidic groups preferably have a pKa less than 5 such that most of the groups are in dissociated form. In an alternative the pKa is less than 4.5 or even less than 4. The pKa may be in a range of 5 to 1 or 5 to 2 or 5 to 3.

Importantly, a cell culturing substrate with any particular shape may now be prepared effectively in one step by mixing the appropriate precursors for forming the bulk modified elastomer and reacting such precursors within a reaction vessel such as that of an injection molding apparatus to form, shape and prepare the surface of a cell culturing substrate such that it can be used for cell culturing without further adaptation. This may also make reproduction of series of such devices easier and more constant.

In some embodiments the one or more acidic groups are phosphorus based acidic groups, sulfur based acidic groups and carboxylic acid groups or a mixture of two or more of these. Phosphoric acid groups and their conjugated bases are preferred over sulphonic acid groups and carboxylic acid groups preferred over phosphoric acid groups. Residues carrying such groups are more easy to use during the bulk modification as during such modification method their precursors having the acidic groups in conjugated base form with metal counter ions and such residues mix better with the other consitiuents to form the elastomer from. The acidic groups may be chosen according to pKa (as specified herein before) needed.

In some preferred embodiments the residues comprise or consist of an aliphatic portion comprising 3 or more carbon atomes and less than 50 carbon atoms, the one or more acidic groups being covalently coupled to the aliphatic portion and the aliphatic portion being covalently coupled to the elatomer bulk. The aliphatic portion may be linear or branched. It can comprise one or more carbon-carbon double or triple bonds or aryl or benzene units. The aliphatic portion can comprise cyclic units such as cyclohexyl or cyclopentyl or other. The aliphatic portion prefearalby is a saturated hydrocarbon portion. The aliphatic portion preferably comprises only carbon and hydrogen atoms.

In some embodiments the aliphatic portion is linear chain coupled to the elastomer bulk at a terminal end. In some embodiments the aliphatic portion further comprises no carbon-carbon triple bonds to increase flexibility of the residue.

In some embodiments the residues within a bulk modified elastomer may be different from eachother each one chosen as defined herein. In some embodiments at least part of the residues maybe bound to the elastomer bul via two covalent bonds. This may for example result from resdieu precursors that have two ethylenic bonds for participating in the modification reaction.

In some embodiments the portion comprise at least 5 carbon atoms and more preferably at least 10 carbon atoms. Preferably the aliphatic portion comprises less than 40 or less than 30 carbon atoms. Linear portions are preferred, but this is not needed perse. The number of carbon atoms of such portion is preferably between 5 and 30 more preferably between 5 and 20 or 5 and 15. The portion and.or chain may have one or more aryl groups. One or more of the acidic groups may be directly attached to an aryl group such as a benzene ring.

In some embodiments the residues are the remainder of unsaturated fatty acid precursors that were covalently bound to the elastomer bulk via reaction of one or more (if present) of their ethylenic groups (carbon-carbon double bonds).

For example the unsaurated fatty acid residues are one or more residues of fatty acids chosen from the group consisting of: myristoleic acid, palmitoleic acid, sapienic acid, oleic acid, elaidic acid, vaccenic acid, linoleic acid, linoeladic acid, α-linolenic acid, arachidonic acid, eicospaentaenoic acid, erucic acid and docosahexaenoic acid

In some embodiments the residues are the remainders of precursors having linear or branched alkyl chains carrying one or more acidic groups and at least one ethylenic group (carbon carbon double bond). Preferably at last one such ethylenic group is a terminal chain ethylenic group. Terminal ethylenic groups may provide increased reactivity as compared to non-terminal ethylenic groups during formation of the elastomer bulk in the manufacturing process. Linear chains with one terminal ethylenic group and one acidic group such as e.g. a carboxyl group are preferred examples. In such case there may be between 5 and 15 carbon atoms in the chain.

In some embodiments the elastomer bulk comprises a silicone or a polydiene backbone. Polybutadiene and polyisorpene are examples of polydienes. Polydimethylsiloxane is an example of a polysioxane (silicone). The silicones have a higher water permeability and are more transparent allowing easier optical inspection of cell culturing.

The residues are covalently bound to the elastomer bulk as a result of a reaction between an unsaturated carbon-carbon bond of a precursor of the residue and a vinyl or hydride functional group of the elastomer bulk. In polydienes such reaction is to vinyl groups while in silicones such binding is usually with silylhydirde functional groups.

The residue concentration in the bulk modified elastomer or the corresponding precursor for the residue in a mixture of precursors for forming the elastomer is in a range of 2.10⁻⁴ to 1.10⁻² mol/kg by weight of the total weight of the elastomer. It has been found that lower concentration causes irreproducible cell culturing properties while higher concentrations are difficult to make in that incomplete mixing of precursors and/or reaction between precursors occurs during manufacture of the bulk-modified material causing irreproducible or deteriorated culturing properties.

In some embodiments the bulk-modified elastomer is obtainable or obtained by mixing a vinyl-functionalized or a hydride-functionalized elastomer or at least one precursor thereof and a residue precursor in a concentration in the range of 2.10⁻⁴ to 1.10⁻² mol/kg by weight of the total weight of the bulk-modified elastomer and heating the mixture. In some embodiments the heating is performed in a reaction vessel having a metal metal oxide inner surface. This is believed to cause that the acidic groups (in conjugated base form) while in the reaction vessel associate at least partly with the oxide surface such that during the polymerisation reactions to form the modified bulk at least part of the acidic groups is available at the surface of the material.

In a further aspect there is provided a cell culturing substrate as claimed hereinwherein the substrate comprises a fluidic device module including a flow channel extending over a membrane comprising the material.

In some embodiments the cell culturing substrate comprises a first maj or surface comprising a first recessed structure defining a first flow channel and a second major surface opposing the first major surface and comprising a second recessed structure defining a second flow channel; wherein the membrane separates the first flow channel from the second flow channel.

In some embodiments of the fluidic devices, the cell culturing substrate of the fluidic device is a monolithic fluidic device.

According to an aspect of the invention there is provided a use of a material having an external surface for culturing cells thereon, the material containing a bulk-modified elastomer having an elastomer bulk and a plurality of residues each comprising one or more acidic groups in free and/or conjugated base form, the plurality of residues being covalently bound to the elastomer bulk such that part of the one or more acidic groups is available on the external surface, the use comprising contacting the external surface with cells to be cultured and a cell growth medium. As explained for the cell culturing substrate embodying this use, such use was not known or obvious while it provides big advantages.

According to yet another aspect there is provided a method of culturing cells, comprising the steps of:
using a cell culturing substrate as claimed in any of the previous claim said, optionally, the substrate being a molded substrate; and
culturing cells directly on said exposed surface of the article.

In some embodiments of the method said celll culturing substrate is one as defined herein.

In some embodiments the cell cultruing method is part of a drug testing method the latter comprising the further step of:
exposing the cultured cells to a drug to be tested through the other of the pair of flow channels of the fluidic device; and
monitoring the response of the cultured cells to the drug to be tested.

### Other non-limiting embodiments

In some embodiments the bulk-modified elastomer is obtained by forming a composition comprising a vinyl-functionalized or a hydride-functionalized elastomer or at least one precursor thereof, a free of saponified unsaturated fatty acid in a range of 0.5 - 5 % by weight of the total weight of the a vinyl-functionalized or a hydride-functionalized elastomer or at least one precursor thereof and a cross-linking catalyst in a mold having a polar inner surface; and bulk-modifying the vinyl-functionalized or the hydride-functionalized elastomer by covalently binding the free or saponified unsaturated fatty acid to the elastomer bulk in said mold by a cross-linking reaction between a vinyl group or a hydride group of the elastomer and an unsaturated carbon-carbon bond of the unsaturated fatty acid to obtain the material.

In some embodiments, there is provided use of a material for culturing cells, the material containing a bulk-modified elastomer having a Shore hardness (DIN EN ISO 868) in a range of Shore00 20 to Shore A 80 and comprising a plurality of fatty acid moieties covalently bound to the elastomer bulk, wherein the carboxylic acid groups of said moieties are available on an external surface of said material to provide said binding, and wherein the bulk-modified elastomer is obtained by forming a composition comprising a vinyl-functionalized or a hydride-functionalized elastomer or at least one precursor thereof, a free of saponified unsaturated fatty acid in a range of 0.5 - 5 % by weight of the total weight of the a vinyl-functionalized or a hydride-functionalized elastomer or at least one precursor thereof and a cross-linking catalyst in a mold having a polar inner surface; and bulk-modifying the vinyl-functionalized or the hydride-functionalized elastomer by covalently binding the free or saponified unsaturated fatty acid to the elastomer bulk in said mold by a cross-linking reaction between a vinyl group or a hydride group of the elastomer and an unsaturated carbon-carbon bond of the unsaturated fatty acid to obtain the material.

It has surprisingly been found that a relatively soft elastomer, i.e. having a Shore hardness in a range of a range of Shore00 20 to Shore A 80 (or Shore A 2-80) as determined using the measurement method specified in the DIN EN ISO 868 standard, when bulk modified with an unsaturated fatty acid as previously disclosed in WO 2019/015988 A1 can act as a biocompatible material onto which cellular material can be directly cultured without the need for an extracellular matrix such as fibronectin when the amount of unsaturated fatty acid is in a range of 0.5 - 5 % by weight (wt%) based on the total weight of the elastomer or its at least one precursor. Without wishing to be bound by theory, it is believed that where the concentration of unsaturated fatty acid is below 0.5 wt%, the density of carboxylic acid groups or carboxylate groups in case of a saponified unsaturated fatty acid is too low to make the external surface of the elastomer onto which the cells are to proliferate sufficiently hydrophilic to achieve suitable proliferation conditions, whereas when this concentration is above 5wt%, some unreacted unsaturated fatty acid may leach from the bulk-modified elastomer and poison the cells adhered to its external surface. Consequently, it is expected that the bulk-modified elastomer has a covalently bound fraction of free or saponified fatty acid groups in a range of 0.5 - 5 wt% based on the total weight of the elastomer given that in this range a full conversion of the cross-linking reaction between the elastomer (or its one or more precursors) and the unsaturated fatty acid in free or saponified form is expected.

The bulk-modified elastomer is chosen to have a hardness in a range of Shore 00 20 to Shore A 80 to ensure that the elastomer is sufficiently flexible to promote cell proliferation, as it for instance is well-known per se that softness of a cell carrier material is essential for growth (and differentiation) of stem cells due to the fact that tissues grown from stem cells want to move, e.g. (heart) muscle, lung, gut and blood vessel tissues. Some organs need to grow in a movable carrier like heart muscles. Hence, where the bulk-modified elastomer material has a Shore A hardness in excess of 80, the material becomes insufficiently stretchable to facilitate the desired cell proliferation directly onto its functionalized surface, in which case the use of a scaffold or extracellular matrix material would be required to provide the developing cell culture with its desired flexibility.

Furthermore, the unsaturated fatty acid used in the bulk modification of the elastomer may be used in free or saponified form, such as for example as a sodium salt. The use of the unsaturated fatty acid in saponified form has the advantage that the risk of catalyst poisoning by the unsaturated fatty acid protons is avoided, whilst it has surprisingly been found that cell culture will also proliferate onto the external surface of a bulk-modified elastomer carrying carboxylate groups with a suitable counter ion such as Na⁺ or K⁺. Hence, this has the further advantage that the saponification does not have to be reversed prior to using the bulk-modified elastomer for cell culturing purposes.

Hence, in this manner a material for cell culturing can be manufactured in a small number of steps, e.g. a single step in some embodiments, by combining the elastomer and the fatty acid in a coating (spin coating, dip coating, spray coating, dispensing) or an injection molding process in which the cross-linking of the elastomer with the fatty acid carbon-carbon double bond can be achieved without significant epoxidation of the fatty acid carbon-carbon double bond due to the limited exposure to ambient oxygen in the spin coating or injection molding process. Consequently, a material is provided in which the elastomer is bulk modified with fatty acid moieties in which the carboxylic acid or carboxylate groups of the (saponified) fatty acid are available to directly bind to harvested cells. Moreover, by controlling the curing process and/or the properties of the mold in which the material is formed, such carboxylic acid or carboxylate groups are also present on an outer surface of the material, thereby providing a substantially homogeneous distribution of carboxylic acid or carboxylate groups on such outer surface, which makes the material particularly suitable for use as a membrane material for a fluidic device, as each cross-section of the material will exhibit the same surface properties, in contrast to membrane materials onto which an anchor for a biocompatible material needs to be grafted or otherwise formed as previously explained. In addition, because typically only a fraction of the elastomer carbon-carbon double bonds are consumed in such a cross-linking reaction, the inventive material retains the elastomeric properties of the elastomer, which adds to the suitability of the inventive material for use in fluidic devices and facilitates the slicing or otherwise cutting of the inventive material for investigative purposes.

Preferably, each of the free or saponified fatty acid moieties is covalently bound to the elastomer bulk through a cross-linking reaction between a vinyl functional group or a hydride functional group of the elastomer and an unsaturated carbon-carbon bond of an unsaturated fatty acid to ensure that the number of carboxylic acid or carboxylate groups available for binding to the biocompatible material can be optimized.

The unsaturated fatty acid may be any suitable unsaturated fatty acid. In an example embodiment, the unsaturated fatty acid is selected from myristoleic acid, palmitoleic acid, sapienic acid, oleic acid, elaidic acid, vaccenic acid, linoleic acid, linoeladic acid, α-linolenic acid, arachidonic acid, eicospaentaenoic acid, erucic acid, undodecanoic acid and docosahexaenoic acid. Linoleic acid is specifically mentioned.

Similarly, the elastomer may be any suitable elastomer. In example embodiments, the elastomer comprises a polybutadiene backbone or a silicone backbone. Where the elastomer comprises a silicone backbone, at least a fraction of the carboxylic acid groups within the silicone backbone may be saponified as saponification of the carboxylic acid groups prior to the cross-linking between the unsaturated fatty acid molecules and the elastomer molecules may be required to prevent the cross-linking catalyst from being deactivated by the protons of the carboxylic acid groups of the unsaturated fatty acid molecules. Equally, the polybutadiene may be cross-linked with a saponified unsaturated fatty acid as the cell culturing properties of the bulk-modified material are not significantly affected by saponification of the unsaturated fatty acid as previously explained.

In a set of embodiments, the composition comprises the free or saponified unsaturated fatty acid in a range of 0.5 - 2 % by weight of the total weight of the a vinyl-functionalized or a hydride-functionalized elastomer or at least one precursor thereof, as it has been found that particularly good cell proliferation is achieved when the amount of free or saponified unsaturated fatty acid to be incorporated into the bulk of the elastomer is within this range. For example, excellent cell proliferation is achieved directly onto the external surface of the bulk-modified elastomer when the composition comprises the free or saponified unsaturated fatty acid in an amount of about 1 % by weight of the total weight of the a vinyl-functionalized or a hydride-functionalized elastomer or at least one precursor thereof.

According to yet a further aspect, there is provided a fluidic device module including a flow channel extending over a membrane, the membrane comprising the inventive material according to any of the herein described embodiments. Such a fluidic device module may comprise a first major surface comprising a first recessed structure defining a first flow channel; a second major surface opposing the first major surface and comprising a second recessed structure defining a second flow channel; with the membrane separating the first flow channel from the second flow channel. In a preferred embodiment, the fluidic device module is a monolithic fluidic device module, which has the advantage that the device module can be manufactured in a small number of processing steps, e.g. a single processing step, by injection molding.

The membrane may comprise a plurality of holes or grooves extending through the membrane. Such holes or grooves preferably have cell-size dimensions to prevent cells from passing through the membrane.

In an embodiment, the first flow channel and the second flow channel are accessible through respective septums, such that the separate flow channels may be individually accessed without cross-contamination risk.

According to yet a further aspect, there is provided a fluidic device comprising the fluidic device module of any of the herein described embodiments and a pair of cover plates to fluidly seal the fluidic device module. The cover plates may be arranged such that one of said cover plates covers the first major surface, thereby sealing the first fluidic channel and the other of said cover plates covers the second major surface, thereby sealing the second fluidic channel. Such a fluidic device can be manufactured in a straightforward manner, as the fluidic device module may be formed in a small number of processing steps as previously explained, and is robust against leakage due to the flexible nature of the fluidic device module.

According to yet a further aspect, there is provided a method of culturing cells, comprising providing a molded article formed from the bulk-modified elastomer material of any of the herein described embodiments, said article comprising the external surface of the material as one of its exposed surfaces; and culturing said cells directly on said exposed surface. In accordance with this method, cells can proliferate directly onto the exposed surface of such an article, e.g. a fluid device module as previously described, without the need for attaching an extracellular matrix such as fibronectin or the like to the exposed surface of the article, thereby significantly simplifying the cell culturing process.

According to still a further aspect, there is provided a drug testing method comprising providing a fluidic device module according to an embodiment; applying harvested cells directly to a surface of the membrane of the fluidic device module; culturing the harvested cells on said surface; forming a fluidic device with the prepared fluidic device module; feeding the cultured cells through one of the pair of flow channels of the fluidic device; exposing the cultured cells to a drug to be tested through the other of the pair of flow channels of the fluidic device; and monitoring the response of the cultured cells to the drug to be tested. Such a drug testing method can be deployed in a simple and straightforward manner, in particular in terms of preparation of the fluidic device to be used in such a drug testing method, thereby providing a significant simplification of existing drug testing methods where such fluidic devices are typically very cumbersome to prepare.

The monitoring of the response of the cultured cells to the drug to be tested may comprise immobilizing and fixating the cultured cells within the fluid device and slicing the fluid device module to obtain a slice for microscopic evaluation, said slice comprising at least a portion of the immobilized and fixated cultured cells. In accordance with the teachings of the present invention, the generation of such slices for microscopic evaluation no longer is critical or subject to variable results due to the fact that the cultured cells are homogeneously distributed across the membrane surface of the fluidic device module owing to the bulk-modification of the elastomer with a free or saponified unsaturated fatty acid as previously explained.

Alternatively, in a preferred embodiment, the monitoring of the response of the cultured cells may be performed within the assembled fluid device using confocal microscopy. This facilitates the real-time monitoring of such responses.

### BRIEF DESCRIPTION OF THE DRAWINGS

Embodiments of the invention are described in more detail and by way of non-limiting examples with reference to the accompanying schematic drawings which are not drawn to scale, wherein:
FIG. 1 depicts the bulk modification of an elastomer with an unsaturated fatty acid according to an embodiment;
FIG. 2 depicts a perspective view of a fluidic device module according to an embodiment;
FIG. 3 depicts another perspective view of a fluidic device module according to an embodiment;
FIG. 4 depicts a perspective view of a fluidic device module according to another embodiment;
FIG. 5 depicts an exploded perspective view of a fluidic device according to an embodiment;
FIG. 6 depicts an exploded perspective view of a fluidic device according to another embodiment;
FIG. 7 depicts an exploded perspective view of a fluidic device according to yet another embodiment;
FIG. 8 depicts an exploded perspective view of a fluidic device according to yet another embodiment;
FIG. 9 is a microscopic image of a cell culture developed on a bulk-modified elastomer according to an embodiment;
FIG. 10; is a microscopic image of a cell culture developed on an elastomer without such bulk modification;
FIG. 11 is a microscopic image of a cell culture on a bulk-modified elastomer according to another embodiment after developing for 1 day;
FIG. 12 is a microscopic image of a cell culture on a bulk-modified elastomer according to another embodiment after developing for 2 days; and;
FIG. 13 is a microscopic image of a cell culture on a bulk-modified elastomer according to another embodiment after developing for 7 days.
FIG. 14 is a microscopic image of a cell culture on a bulk-modified elastomer according to another embodiment after developing for 4 days.
FIG. 15 is a microscopic image of a cell culture on a bulk-modified elastomer according to another embodiment after developing for 4 days.
FIG. 16 is a graph showing measured contact angles for substrates with silicone having 1 wt % linoleic acid modification. Vertical axis is contact angle in degrees. 4 Sets, each of two bars, represent average contact angle for the 4 batches of substrates. The left bar of each set represents contact angle at pH of 4 and the right bar of each set represents the contact angle at pH of 10. The left most set of bars represents non modified silicone while the others represent modified substrates that were used in the cell culture examples 1, 2 and 3 respectively.

### DETAILED DESCRIPTION OF THE EMBODIMENTS

It should be understood that the same reference numerals are used throughout the Figures to indicate the same or similar parts.

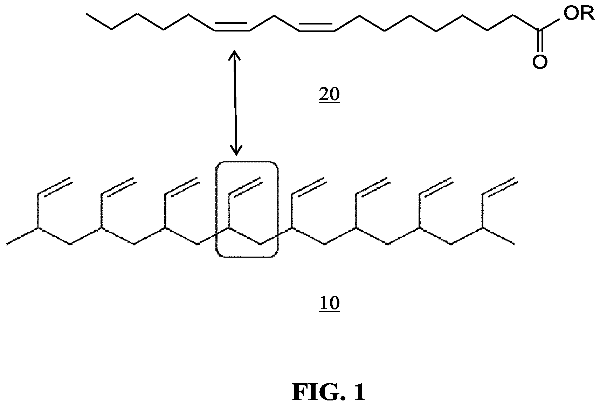

FIG. 1 depicts a reaction between ethylenic bonds of a precursors of residues 20 having and the ethylenic bond of a polymer backbone 10 for producing a bulk-modified elastomer material according to some embodiments of the present invention. In this example reaction is between a fatty acid precursor residue linolenic acid and a vinyl-functionalized elastomer 10 (from here on simply referred to as elastomer) in the form of polybutadiene. Such reaction may be catalyzed with appropriate catalyst such as for example a peroxide. Although in this Fig. 1 polybutadiene 10 is shown to react with linoleic acid in saponified form with R is e.g. alkali metal such as lithium, sodium or potassium ion, it will be clear that other ethylenic bond precursor residues and other ethylenic bond backbone polymers may be used in such reaction type.

It has been found that such a reaction can provide a bulk-modified elastomer in which the carboxylic acid groups of at least a fraction of the unsaturated fatty acid molecules 20 having engaged in the reaction with the elastomer 10 are presented on an external surface of the bulk-modified elastomer, such that these carboxylic acid groups are available for stabilizing cell cultures directly on the external surface of the bulk-modified elastomer 10. Thus, a step of binding a protein to such carboxylic acid groups to facilitate the cell stabilization is no longer needed and this saves considerable time and effort and opens new opportunities for defining cell culture substrates including the material. For example, the formation of the elastomer and shaping of the elastomer may now be done in one step using an appropriate reaction vessel. In preferred examples such reaction vessel is the mold for example of an injection molding apparatus. It is noted that the example of Fig. 1 is just one type of ethylenic bond chemistry that can be used in such process. As will be described hereinbelow a reaction based on addition of silyl hydrides over ethylenic bonds in the presence of a catalyst can also be used as will be explained in more detail herein below.

Preferably the elastomer 10 is soft or flexible to facilitate cell-induced movement. For this reason, the elastomer 10 typically may have a Shore hardness as determined in accordance with the DIN EN ISO 868 standard ranging from Shore 00 20 (i.e. Shore A 2) to Shore A 80, i.e. a Shore A hardness of 2-80. Suitable elastomers include polyenes such as polybutadiene and silicones.

The unsaturated fatty acid 20 may be a free unsaturated fatty acid, in which case R = H or may be a saponified fatty acid, in which case R = Na⁺ or K⁺ (and the COO group is a COO⁻ or carboxylate anionic group) for example. Such saponification may be achieved in any suitable manner, e.g. using a concentrated NaOH or KOH solution. As such saponification reactions are well-known per se, this will not be explained in further detail for the sake of brevity only.

Importantly, the saponification of the unsaturated fatty acid residues in the bulk modified elastomer s does not need to be reversed as it has been surprisingly found that cell proliferation on the external surface of the bulk-modified elastomer 10 also takes place when this surface is functionalized with saponified carboxylic acid groups, i.e. carboxylate groups having a counter ion such as a Na⁺ or K⁺ counter ion, as will be explained in further detail below.

The presence of the carboxylic acid or carboxylate groups on the external surface of the bulk-modified elastomer 10 may be considered surprising given that the elastomer 10 can be non-polar in character, in particular when the elastomer 10 is dissolved in a non-polar solvent. In such a non-polar environment, the unsaturated fatty acid molecules 20 tend to form micelles in which the carboxylic acid moieties of these molecules are turned inwardly within such micelles, which typically leads to such carboxylic acid moieties ending up within the bulk of the modified elastomer. In addition, it is well-known per se that the carbon-carbon double bonds of such unsaturated fatty acids 20 are prone to rapid epoxidation in the presence of oxygen, which reduces the ability of such compounds to engage in cross-linking reactions with the elastomer 10.

Embodiments of the present invention are based on the insight that if a reaction mixture including the elastomer 10 and the unsaturated fatty acid 20 in saponified form is brought into contact with a polar surface, this promotes orientation of the carboxylic acid groups of the fatty acid molecules along such a polar surface such that an external surface of the bulk-modified elastomer material exhibits a high density and homogeneous distribution of these carboxylic acid groups. Moreover, it has been found that when bulk modifying the elastomer 10 in this manner in an injection molding process at elevated temperatures, e.g. temperatures in a range of 120-240°C, epoxidation of the carbon-carbon double bonds of the unsaturated fatty acid molecules 20 does not significantly interfere with the bulk modification of the elastomer 10, i.e. does not significantly inhibit the reaction between the unsaturated fatty acid molecules 20 and the elastomer 10. Without wishing to be bound by theory, it is believed that in such injection molding processes, molecular oxygen is largely absent from the reaction mixture within the injection molding apparatus, thereby suppressing the unwanted epoxidation of these carbon-carbon double bonds.

In order to obtain an elastomer 10 onto which cells can be directly cultured, the elastomer 10 is bulk-modified with an amount of the unsaturated fatty acid 20 in free or saponified form in a range of 2.10⁻⁴ to 1.10⁻² mol/kg by weight of the total weight of the elastomer 10 or the total weight of the one or more precursors of the elastomer 10. If a smaller amount of the unsaturated fatty acid 20 in free or saponified form is used, it has been found that cells will not substantially proliferate onto the bulk-modified elastomer 10, which may be due to the surface of the bulk-modified elastomer 10 onto which the cells are seeded being too hydrophobic in nature. On the other hand, if a larger amount of the unsaturated fatty acid 20 in free or saponified form is used, the cells also largely fail to proliferate on the surface of the bulk-modified elastomer 10 and/or the proliferation results differ from experiment to experiment such that they are not reproducible, which may be caused by unreacted unsaturated fatty acid 20 in free or saponified form leaching from the bulk modified polymer and poisoning the cell culture. Moreover, it has been found that when the amount of unsaturated fatty acid 20 in free or saponified form is in the aforementioned range, the flexibility or hardness of the unmodified elastomer 10 is not significantly altered by the bulk modification with the unsaturated fatty acid 20 in free or saponified form. Furthermore, transparency to at least optical radiation such as e.g. visible light of the elastomer becomes less for example because of increased scattering due to opaqueness when higher concentrations are used. This may impair the use of the cell culturing substrate for optical inspection of the cell proliferation or culturing.

Although the example of Fig. 1 was described for a polydiene type bulk elastomer, other elastomers may be used. Preferably such elastomers also have the desired Shore hardness.

Thus, elastomers that can be used are for example, a homopolymer, a copolymer, a block copolymer, a terpolymer, a block terpolymer and so on. A particularly suitable elastomer may be selected from polyenes such as polybutadiene, or isoprene or neoprene. Where polyenes are used, they may be made using any suitable polymerization process after which the coupling as described with reference to Fig/ 1 may be performed. Particularly preferred is a polybutadiene formed in a Nd or Li-catalyzed polymerization reaction, as it is well-known per se that such polybutadiene has a low degree of branching (typically below 5%) and a low degree of polydispersity (Mw/Mn) of around 2. Li-catalyzed polybutadiene is particularly preferred due to its high degree of 1,2-vinyl content (about 11%). However, other types of polybutadiene, e.g. polybutadiene obtained from a Co, Ni or Ti-catalyzed polymerization reaction may be used instead. Where a polyene such as polybutadiene is used as the elastomer 10, the cross-linking reaction between the elastomer 10 and the unsaturated fatty acid 20 may be catalyzed using a peroxide catalyst. In another advantageous embodiment, a Li-catalyzed polybutadiene is used having a degree of branching in a range of 5-15% as it has been found that when the degree of branching of the polybutadiene is in this range, the reactivity of the polybutadiene with the unsaturated fatty acid 20 is improved. It is noted for the sake of completeness that it is well-known per se how to control the degree of branching when synthesizing polybutadiene such that this will not be further explained for the sake of brevity only.

Another class of desired elastomer is silicones (polysiloxanes) as these are suitable for injection molding processing. Such silicones may comprise a Polydimethylsiloxane (PDMS) backbone including vinyl moieties to facilitate cross-linking through Pt-catalyzed addition reactions, e.g. with (poly methyl) hydrogen siloxanes. Instead of or in addition to such cross-linking with (poly methyl) hydrogen siloxanes, the vinyl-functionalized PDMS backbone may be reacted with unsaturated fatty acids 20.

Such elastomers in the form of silicones may be formed by a cross-linking reaction between a vinyl-functionalized linear or branched silicone monomer or oligomer, e.g. a T-branched or Q-branched silicone monomer or oligomer and a linear hydride-functionalized silicone monomer or oligomer, between a hydride-functionalized linear or branched silicone monomer or oligomer, e.g. a T-branched or Q-branched silicone monomer or oligomer and a linear vinyl-functionalized silicone monomer or oligomer or mixtures of vinyl-functionalized and hydride-functionalized linear or branched silicone monomers or oligomers, e.g. a T-branched or Q-branched silicone monomer or oligomer and/or a mixture of linear hydride-functionalized and vinyl-functionalized silicone monomer or oligomer.

For example, the two-component silicone may be formed by a cross-linking reaction between a linear component 1 and a linear component 2, which each may correspond to the general formula below:

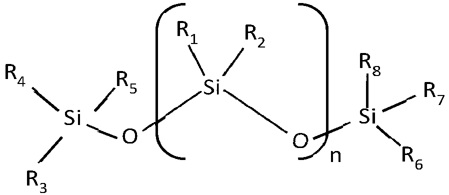

In component 1, R1 and R2 are individually selected from C1-C3 alkyl,R3-R8 are individually selected from C 1-C3 alkyl and vinyl, with the proviso that at least one of R3-R5 and at least one of R6-R8 is vinyl. Preferably, at least three of R3-R8 are vinyl. In all embodiments, n may be in the range of 100 - 200,000. In a specific embodiment of component 1, each of the groups R1-R8 that is an alkyl group is a methyl group, i.e. the component 1 is vinyl-functionalized PDMS having terminal vinyl groups.

In component 2, R1 is hydrogen, R2 = C1-C3 alkyl and R2-R8 are individually selected from C1-C3 alkyl or hydrogen, with the proviso that at most one of R3-R5 and at most one of R6-8 is hydrogen, and and n may have any suitable value, such as n = 3-1,000 or more specifically n = 3-10. In a specific embodiment of component 2, none of the groups R3-R8 are hydrogen. In another specific embodiment of component 2, each of R2-R8 are methyl.

Component 2 typically acts as a cross-linking agent for component 1. Such cross-linking reactions, which are typically Pt-catalyzed, are well-known per se, see for example WO 2009/147602 A2 and are therefore not explained in further detail for the sake of brevity.

Bulk modifying such silicones may be done by forming the elastome in the presence of an unsaturated fatty acid 20, the covalent bond between the silicone and the unsaturated fatty acid 20 may be formed by a reaction between a hydride functional group of the (cross-linked) silicone and the unsaturated fatty acid 20 as suggested in the below reaction mechanism:

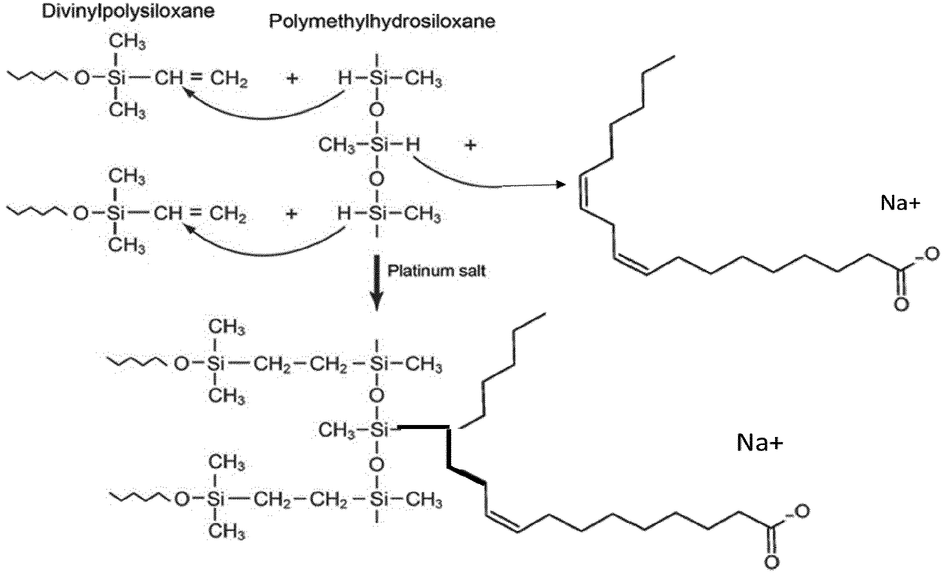

In order to prevent catalyst inhibition by the protons of the carboxylic acid groups of the unsaturated fatty acids 20 during such cross-linking reactions, the unsaturated fatty acid may be used in a saponified form in the cross-linking reaction with a silicone elastomer, e.g. as the sodium salt of such an unsaturated fatty acid. In some emobdiments if deisred, the reaction product (the bulk modified elastomer) subsequently may be treated with a protic acid, e.g. HCl or the like, in order to reinstate the carboxylic acid groups on the surface of the bulk-modified elastomer, but, this is not necessary in case the bulk-modified elastomer is to be used for cell culturing directly on its surface, given that it has been demonstrated that cells will also proliferate when the polar surface groups are carboxylate anions, i.e. saponified carboxylic acid groups. Any silicone elastomer having free silylhydride groups present for reacting with the ehtylenic bond carrying residue precursor may be used for such bulk modification of the elastomer.

It is noted that elastomer formation does not need to have commenced before performing the modification reaction. This may be done in some embodiments, but preferably the modification is performed at least partly during the elastomer formation by mixing the appropriate amounts of elastomer formation components and modification precursor residues.

Alternatively, a silicone backbone such as a (poly methyl) hydrogen siloxane backbone may be crosslinked with rubber-like polymers such as polybutadiene and polyisoprene, with the unsaturated fatty acid being incorporated in such a crosslinked product. This for example may be done to tune the water permeability of the final material as silicones typically have a rather open structure whilst such rubber-like polymers have a rather closed structure, such that the openness, i.e. the water permeability, of the crosslinked product can be tuned by the ratio of silicone and rubber-like polymer therein. In this manner, the properties of the material according to this embodiment may be optimized for cell/protein interaction to build good scaffolds. For example, where the elastomer is a silicone-based, e.g. PDMS-based, a material that is highly permeable to water and other compounds (e.g. drugs) is obtained that facilitates maximized perfusion of these compounds into the cells on the membrane of the fluidic device module 100. However, where real-time monitoring of drug consumption by cells is desirable, a polyene-based material such as a polybutadiene-based material that is non-permeable to water and drugs may be preferable, as the rate of perfusion of these compounds to the cells can be controlled using the density of apertures through the membrane.

In an embodiment, the polysiloxane may be formed from a multi-component starting material as previously explained in order to prevent premature cross-linking of the polysiloxane. Such multi-component starting materials are well-known per se; for example, such multi-component starting material kits are marketed by Wacker Chemie AG from Munich, Germany under the tradename Elastosil.

Linoleic acid has been used as the precursor for the acidic group carrying residue with which the bulk modified elastomer is modified. It should be understood that this is by way of non-limiting example only as other acdic group carrying residues may be used as will described herein below. Advantages described herein before obtained with linoleic acid will also be achieved with the further residues and their precursors below unless otherwise indicated.

Thus, for example regarding unsaturated fatty acid 20, any suitable unsaturated fatty acid may be used for the purpose of reacting it with the elastomer 10. For example, the unsaturated fatty acid 20 may be selected from myristoleic acid, palmitoleic acid, sapienic acid, oleic acid, elaidic acid, vaccenic acid, linoleic acid, linoeladic acid, α-linolenic acid, arachidonic acid, eicospaentaenoic acid, erucic acid, undodecanoic acid and docosahexaenoic acid.

More general in the context of the present application, where reference is made to an unsaturated fatty acid 20 in relation to the example of Fig. 1, it is to be understood that this is intended to refer to any organic compound comprising one or more acidic groups either in free or conjugated base form and comprising at least one carbon-carbon double bond.

In general the residues in the bulk modified elatomer result from reaction of a residue precursor (that is used for the modification of the bulk modified elastomer) with the elastomer bulk or its precursors. Fatty acids are in this sense only one type of such residue precursors. Others exist and may be used as long as they have at least one carbon-carbon double bond and one or more acidic groups, which need to be in conjugated base form when used in conjunction with the silicone chemistry. In the polydiene chemsitry such conjugated base form is not needed per se.

In some preferred embodiments the residue precursors comprise or consist of an aliphatic portion comprising 3 or more carbon atomes and less than 50 carbon atoms, and at least one carbon-carbon double bond, where the one or more acidic groups are covalently coupled to the aliphatic portion. The aliphatic portion may be linear or branched. It can comprise one or more (e.g. 2, 3 or 4) carbon-carbon double or triple bonds (preferably only double bonds) or aryl or benzene units. The aliphatic portion can comprise cyclic units such as cyclohexyl or cyclopentyl or other. The aliphatic portion preferably comprises only carbon and hydrogen atoms. The aliphatic portion may be a saturated hydrocarbon portion. In some embodiments the aliphatic portion further comprises no carbon-carbon triple bonds to increase flexibility of the residue.

In some embodiments the aliphatic portion comprises at least one terminal carbon-carbon double bond (H₂C=CH-R, with R rest of the aliphatic portion), preferably the portion is entirely linear with at least one (fore example only one) acidic group attached at the other terminal end of the portion.

In some embodiments a mixture of precursor residues may be used to arrive at a bulk modified elastomer with at least part of its residues mutually different. Each of the precursor residues may be chosen as defined herein. In some embodiments at least part of the aliphatic protion of the residue precursors includes at least 2 carbon carbon double bonds. In this way the residues in the bulk modified elastomer may be covalently bound to the elastomer bulk with two or more bonds. As a consequence, polymer chains of the elastomer bulk may become cross-linked via the residue.

In some embodiments the portion of the precursor residue comprise at least 5 carbon atoms and more preferably at least 10 carbon atoms. Preferably the aliphatic portion comprises less than 40 or less than 30 carbon atoms. The number of carbon atoms of such portion is preferably between 5 and 30 more preferably between 5 and 20 or 5 and 15. The portion and/or chain may have one or more aryl groups. One or more of the acidic groups may be directly attached to an aryl group such as a benzene ring.

In some embodiments the residue precursors are unsaturated fatty acids and the residues the remainder of such unsaturated fatty acid precursors that were covalently bound to the elastomer bulk via reaction of one or more (if present) of their ethylenic groups (carbon-carbon double bonds).

For example the unsaturated fatty acid residues are one or more residues of fatty acids chosen from the group consisting of: myristoleic acid, palmitoleic acid, sapienic acid, oleic acid, elaidic acid, vaccenic acid, linoleic acid, linoeladic acid, α-linolenic acid, arachidonic acid, eicospaentaenoic acid, erucic acid and docosahexaenoic acid

In some embodiments the residues are the remainders of precursors having linear or branched alkyl chains carrying one or more acidic groups and at least one ethylenic group (carbon carbon double bond). Preferably at last one such ethylenic group is a terminal chain ethylenic group. Terminal ethylenic groups may provide increased reactivity as compared to non-terminal ethylenic groups during formation of the elastomer bulk in the manufacturing process. Linear chains with one terminal ethylenic group and one acidic group such as e.g. a carboxyl group are preferred examples. In such case there may be between 5 and 15 carbon atoms in the chain.

Thus, the residue may have an aliphatic part comprising at least 3 carbon atoms to which one or more acidic groups are covalently bound. The corresponding precursor for such residue includes at least one carbon-carbon double bond in its aliphatic part. Preferably the aliphatic part comprises 5 or more, 7 or more, 9 or more 11 or more carbon atoms. Preferably the aliphatic part comprises less than 30 or less than 20 or less than 15 carbon atoms. The aliphatic part of the residue or precursor may be linear or branched, with linear preferred. It may have cyclic structures. The aliphatic tail may terminate in a nonaromatic ring structure, e.g. a five-membered or 6-membered cyclopentyl or cyclohexyl group, which may itself contain at least one double bond. A non-limiting example of an organic compound intended to fall within the above definition of an unsaturated fatty acid 20 is retinoic acid.

The aliphatic part may have one, two, three or four ethylenic bonds. Preferably there are less than 3. More preferably there is only 1. At least one ethylenic bond is substituted with at least two hydrogen atoms. More preferably at least one ethylenic bond is substituted with three hydrogen atoms. Such ethylenic bonds typically show increased reactivity in reacitons described herein with higher reactivity for the latter mentioned one.

The acidic groups carried by the aliphatic parts are preferably connected to a saturated carbon atom of the aliphatic part. Acidic groups may be carboxylic acid groups and their carboxylate groups (conjugated base groups), phosphorus comprising acidic groups such as phosphoric acid groups and the corresponding phosphates and hydrogen phosphates, phosphorous acid groups and the corresponding conjugated bases and/or sulfur comprising acidic groups such as sulponic acid groups and the corresponding sulphonates.

A phosphoric acid group is a phosphorus oxoacid in which each phosphorus atom is in the oxidation state +5, and is bonded to four oxygen atoms. Two or more of these PO4 units (tetrahedra) may be connected by shared single-bonded oxygens, forming linear or branched chains with at least one of the oxygen atoms bonded to the rest of the residue. The single-bonded oxygen atoms that are not shared are completed with acidic hydrogen atoms or can accept protons. The general formula of a phosphoric acid group is Hₙ₊₁₋₂ₓPₙO₃ₙ₊₁₋ₓ, where n is the number of phosphorus atoms and x is the number of fundamental cycles in the molecule's structure, between 0 and (n+2)/2. There is one bond reserved for bonding to the residue, for example the aliphatic part of such residue. Preferably the acidic groups comprise orthophosphoric acid groups or polyphosphoric acid groups R-O(O=POHO)ₙ-H with n is 1 for orthophosporic acid and n>2 for polyphosporic acid while R represents the rest of the residue to which the group is bound. For example there may be Phosphoric acid groups are well known in the art and will not be further described in detail. Further definitions can be found in many textbooks on organic chemistry.

Any of the conjugated bases of the acidic groups such as e.g. the carboxylates, phosphates, hydrogen phosphates sulfonates etc. preferably are associated with or saponified with metal counter ions such as those of the alkali metals (e.g. lithium, sodium and potassium) and alkaline earth (e.g. barium and calcium) metals.

Many other compounds in addition to the example compounds explicitly mentioned in the present application will be immediately apparent to the skilled person.

There may be one acidic group per residue or precursor of such residue. In such case the molalities of residues in the bulk modified elastomer or of the precursors used to prepare such bulk modified elastomers also reflect the molalities of acidic groups present within the bulk modified elastomer. To calculate the final molality of acidic groups from the the molality of the residue, the latter needs to be multiplied by the number of acidic groups per residue. It may be beneficial to have more than one acidic group per residue when a molality of such groups within the bulk modified elastomer is needed that is higher than the 1.10⁻² mol residue / kg bulk modified elastomer without substantially being bothered by problems associated with an amount of residue higher than this range limit as explained herein before.

As disclosed herein before, the residue concentration in the bulk modified elastomer or the corresponding precursor for the residue in a mixture of precursors for forming the elastomer is preferably in a range of 2.10⁻⁴ to 2.10⁻² mol/kg by weight of the total weight of the elastomer or the total weight of the one or more precursors of the elastomer. For silicone type elastomers this residue concentration range preferably is 2.10⁻⁴ to 1.10⁻² mol/kg. This largely avoids washing of the substrate to remove unreacted precursor before use. The residue molality range may be 2.10⁻⁴ to 2.10⁻³ mol/kg for a low concentration of acidic groups. It may be 1.10⁻³ to 5.10⁻³ for a higher concentration of acidic groups. The cell proliferation for different cell lines may require different concentrations within the ranges.

The molalities disclosed herein may be translated into weight percentages when considering the molar weights of the residues or precursors for residues using commonly known calculations.

It is noted that when using the silylhydride addition over vinyl bond chemistry for forming the silicone based elastomers, the residue precursors are used with their acidic groups in conjugated base form. For example, they are saponified. As such they are added as salts of the alkali or alkaline-earth metals. This avoids contamination of the Pt catalyst used for the aforementioned chemistry. This is however not needed for the polydiene type modification as in that chemistry acid insensitive catalysts are used.

An example synthesis procedure for producing the bulk-modified elastomer from a polyene such as polybutadiene is given below (synthesis example 1).

### Synthesis example 1

Polybutadiene chunks as obtained from Lanxess AG, Cologne, Germany are inserted into a compounder or mix extruder. The chunks contain a peroxide such as Dicumylperoxide as a catalyst. An amount of linoleic acid (60-74% concentrated as obtained from Aldrich Chemistry) in a range of 1-5 wt% by weight of the polybutadiene chunks is mixed into the polybutadiene bulk by kneading at a temperature in the range of 110-150°C. The resulting extruded mixture is injected into a stainless steel mold defining a fluidic device and having an oxidized inner surface contacting the extruded mixture for 3-10 minutes at a temperature in a range of 150-200°C to yield a monolithic fluidic device module of a polybutadiene rubber cross-linked with the linoleic acid.

An example synthesis procedure for producing the bulk-modified elastomer from a silicone rubber is given below (synthesis example 2).

### Synthesis example 2

A 4.848 g sodium linoleate solution in a solution of water and isopropylalcohol (1: 1 ratio of the solvents by weight) such that the weight fraction of the sodium linoleate in solution is 30% by weight is mixed into silicone component A (240g) of Elastosil LR 3040 from Wacker Chemie AG and vigorously stirred under vacuum conditions at room temperature to evaporate the water from the composition. The resultant mixture is mixed with component B (240g) of Elastosil LR 3040 from Wacker Chemie AG (including Pt-catalyst) and fed into an injection molding apparatus where the resultant mixture is injected into a stainless steel mold having an oxidized inner surface contacting the resultant mixture and defining a fluidic device module and molded for 10-60 seconds at a temperature in a range of 150-200°C to yield a monolithic fluidic device module of a silicone rubber cross-linked with the sodium linoleate in which the sodium linoleate is present in the polymer matrix in an amount of 1% by weight relative to the weight of the polymer matrix (based on starting material weights). Optionally, the molded product may be subsequently dropped in an aqueous HCl solution (pH 2-4) to convert at least the sodium carbonate groups at the surface of the molded product into free carboxylic acid groups.

### Synthesis example 3

Identical to the example 2, but using sodium undecanoate in the appropriate amount to arrive at a same molality as for the sodium linoleate of example 3.

To further demonstrate the bulk modification of the elastomers in synthesis examples 1 and 2, a block of each elastomer after reaction with the linoleic acid was molded in a polar mold as previously explained and subsequently sliced with a microtome at -40°C to obtain an internal slice of the modified elastomer, i.e. a slice in which none of its major surfaces were external surfaces of the molded elastomer block.

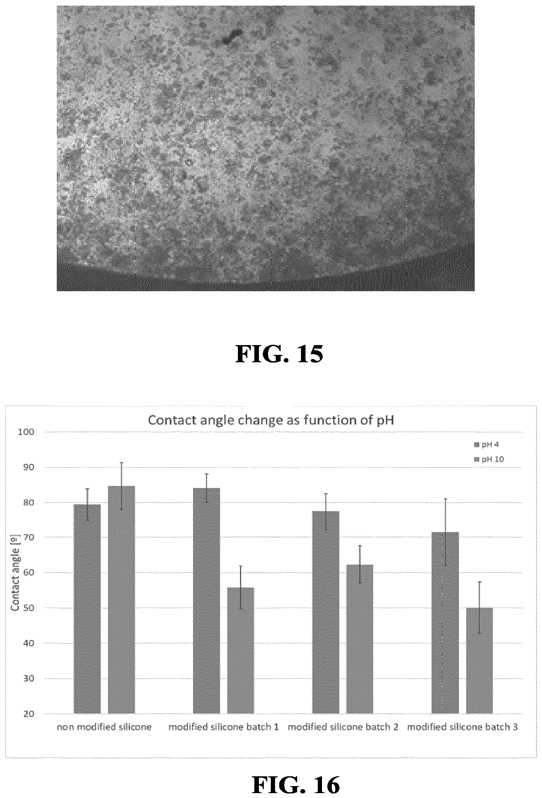

To show that part of the modifying residue acidic groups are actually available at the surface of the elastomers molded, these internal slices were subsequently brought into contact with a 2 µl droplet of water of different pH (pH 4, pH 7 and pH 10) for 3 minutes after which the contact angles of the water droplets with these internal slices were measured. The same procedure was repeated with unmodified elastomers to compare the measured contact angles of an internal slice of the linoleic acid modified elastomers against the measured contact angles of an internal slice of the corresponding unmodified elastomer. By way of example the results for the silicone-based elastomer are shown in the graph in FIG. 16.

As can be seen from these graphs, the contact angle for the unmodified silicone is largely insensitive to the pH of the droplet. Without wishing to be bound by theory, the small increase in contact angle measured for the unfunctionalized silicone with increasing pH may be result of a pH dependence on the hydrogen bonding between the water molecules and silanol moieties in the silicone. For the modified silicone batches used for the different cell type culturing described herein below, the contact angle at pH=4 is substantially lower than that at pH=10.

The behavior (not shown) observed for modified silicones is the same regardless of the form (free or conjugated base) of the surface available carboxylic acid groups.

Further, the bulk modification is evident from the same behavior that is observed at a cutting surface prepared by cutting a batch of a substrate in two halves.

Thus, both internal slices (obtained after cutting) and external surfaces of the polybutadiene crosslinked with linoleate showed a strong pH dependency of the measured contact angle with decreasing contact angles being measured with increasing pH.

While not shown here, similar contact angle behavior is observed for the modified polybutadiene, whereas the measured contact angles of the unmodified polybutadiene are largely pH independent.

The strong pH dependency of the measured contact angle for the bulk and surface of the functionalized elastomers clearly demonstrates the presence of carboxylic acid groups within the bulk and at the surface of the modified elastomers. This can be understood as follows. At lower pH, such carboxylic acid groups remain largely protonated, thereby minimizing the surface charge of the internal slices of the elastomers. With increasing pH, this surface charge increases due to increased deprotonation of the carboxylic acid groups, which decreases the contact angle of the water droplets with the surface of these internal slices. On the other hand, where such carboxylic groups are not present at the surface exposed to the water droplets, such as in the unmodified elastomers, the change in pH of the water droplets does not cause a change in the surface charge, such that the measured contact angle of the water droplets of different pH with such a surface is far less sensitive to these pH changes.

The pH of droplets to be used for the contact angle discrimination method are dependent on the pKa value of acidic groups used in a substrate. In the linoleic acid group case pKa is around 4.7 and hence the low pH droplet should have a pH below 4.7 and the high pH droplet a pH around 2 pH units higher than the pKa to cause full dissociation in the droplet. Those skilled in the art will be able to choose the correct conditions for particular acidic groups with a certain pKa.

A further method to check availability of surface acidic groups (carboxylic acid groups) was performed. To this end all slabs or slices were treated with HCl as described herein to revert any accessible conjugated base of acidic groups to its acid form. The resulting dried slabs were coupled to fluorescent labelled cadaverine by reacting the NH₂ group of the cadaverine to the acidic group using the well-known coupling chemistry of EDC/NHS. A comparison of treatment of the modified elastomers and a non-modified elastomer clearly shows increased Fluorescence for the latter with quite homogenous spatial greyscale and absence of fluorescence for the non-modified elastomer. Such staining test can be used to show the presence of surface accessible acidic groups such as carboxylic acid groups.

Bulk presence of the acidic groups was investigated using IR measurements in both reflective and transmissive mode as well as with elemental analyses.

The completion of or progression of polymerization reactions for forming the modified elastomers was followed using IR and Raman spectroscopy form which conversion rates and states were determined. For example by looking at reduction of absorption of vibrations of the ethylenic bonds of the precursors of the modifying residues.

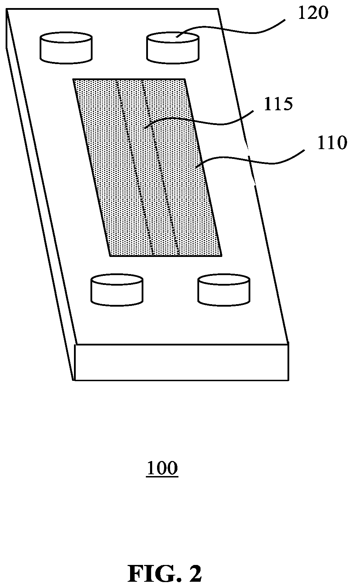

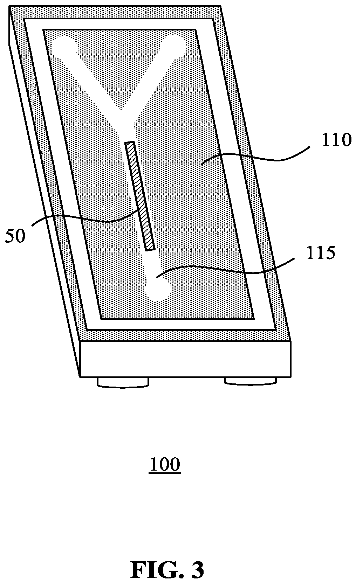

A bulk-modified elastomer may be molded in any suitable form to produce an article or cell culturing substrate onto which cells may be directly cultured. Such articles may take any suitable shape. In a particular embodiment, the mold used in such an injection molding process may be shaped in the form of a fluidic device module such that a monolithic fluidic device module may be formed in a single step injection molding process. Due to the high density and homogeneous distribution of carboxylic acid groups or carboxylate groups on the external surface(s) of such a monolithic fluidic device module, such a device module can be used a straightforward manner as a cell-culturing substrate. The mold may be made of or have an inner surface coated with any suitable polar material, such as stainless steel, a metal or a metal oxide such as aluminium oxide in order to achieve the desired orientation of the carboxylic acid or carboxylate groups of the bulk-modified elastomer on at least one of its external surfaces. A typical example of a fluidic device module 100 is schematically depicted in FIG. 2 and FIG. 3, in which perspective views of opposing major surfaces of the fluidic device module 100 are schematically depicted. The fluidic device module 100 may comprise a membrane 110 over which at least one flow channel 115 extends. Preferably, the fluidic device module 100 comprises opposing flow channels spatially separated by the membrane 110. At least the membrane 110 is made of the bulk-modified elastomer 30 although in a preferred embodiment the entire fluidic device module 100 is made of the bulk-modified elastomer 30, thereby yielding a monolithic fluidic device module 100.

The fluidic device module 100 may comprise one or more septums 120 through which such flow channels 115 may be accessed. Each flow channel 115 preferably is accessible through a dedicated set of septums 120, such that cross-contamination between multiple flow channels 115 is avoided. As explained above, an exposed surface of the fluidic device module 100, e.g. the membrane 110, may be used to directly bind a cell culture, such that cells may be cultured (proliferated) on the biocompatible surface.

The membrane 110 may comprise a plurality of holes or grooves such that liquid nutrition and solutions with compounds such as potential drugs may reach both sides of the membrane and cells/tissue, which holes or grooves may be formed in any suitable manner, e.g. through laser cutting. Such holes or grooves in the membrane 110 of the fluidic device module 100 preferably are in the same order as the typical diameter of the cells to be immobilized on the membrane 110, with the pitch between such holes or grooves not being particularly critical.

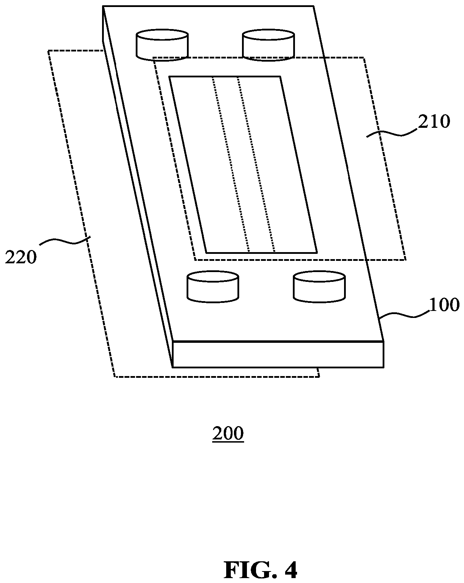

A fluidic device 200 as schematically depicted in FIG. 4 may be formed from such a fluidic device module 100 by the provision of a pair of cover plates 210, 220, which cover plates fluidly seal the fluidic device module 100 such that fluids passed through the one or more flow channels 115 of the fluidic device module 100 cannot leak out of the fluidic device 200.

In an embodiment, the cover plates 210, 220 are arranged such that the cover plate 210 covers a first major surface of the fluidic device module 100, thereby sealing a first fluidic channel 115 of the fluidic device module and the other cover plate 220 covers a second major surface of the fluidic device module 100, thereby sealing a second fluidic channel 115 of the fluidic device module. The cover plates 210 and 220 may be made of any suitable material, e.g. glass or a plastic material. Preferably, the cover plates are stretchable and flexible to ensure a good fit with the fluidic device module 100 whilst retaining the flexibility of the fluidic device module 100 in the fluidic device 200. Such a fluidic device 200 may be deployed as an organ on chip as will be readily understood by the skilled person.

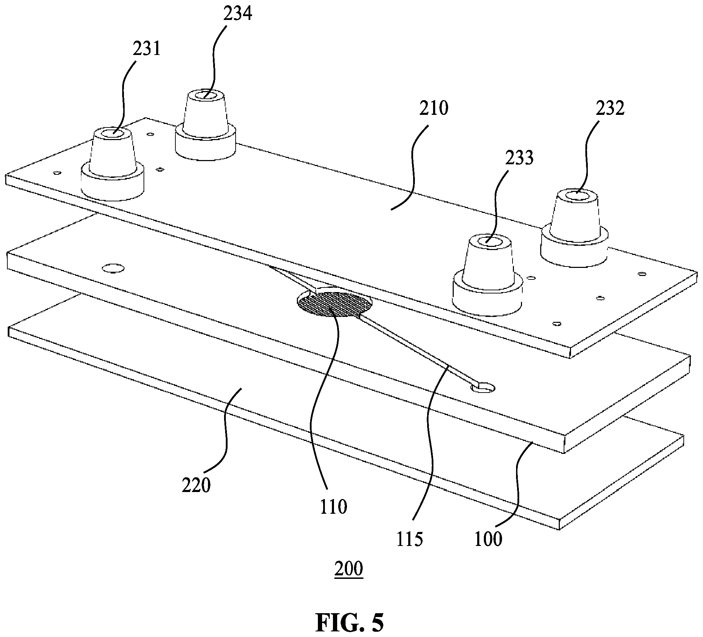

FIG. 5 schematically depicts an example embodiment of a fluidic device 200 in which the device comprises a fluidic device module 100 according to the present invention in between two rigid cover plates 210 and 220. The upper plate 210 comprises a first inlet 231 and a first outlet 232 in fluid connection with a lower channel (not visible) underneath the membrane 110 in the fluidic device module 100 such that the membrane 110 is in fluid contact with a fluid passing through this lower channel. The upper plate 210 further comprises a second inlet 233 and a second outlet 234 in fluid connection with an upper channel 115 over the membrane 110 such that the membrane 110 is in fluid contact with a further fluid passing through this upper channel 115. For example, the fluid passing through the lower channel may comprise drugs to be tested on the cell cultures attached to the membrane 110 as explained in more detail below, which can reach the cell cultures through the membrane 110 owing to the fact that the membrane 110 is made porous, e.g. by laser drilling holes or grooves in the membrane 110 as previously explained. Alternatively, such holes or grooves may be formed in the membrane 110 when forming the fluidic device module 100 in the polar mold. This has the advantage that a regular pattern of such fluid passages (pores) may be formed through the membrane 110. The further fluid passing through the upper channel 115 may be in direct contact with such cell cultures and provide such cell cultures with nutrients. The inlets 231, 233 and the outlets 232, 234 in some embodiments are septa through which fluids may be hydraulically pumped over the membrane 110 through the aforementioned channels, e.g. by pressing the septa. Alternatively, the inlets 231, 233 and the outlets 232, 234 may be ferrules to which tubing may be attached, e.g. clamped, which for instance is useful when the fluidic device 200 is used as a well plate or the like in a lab on chip system.

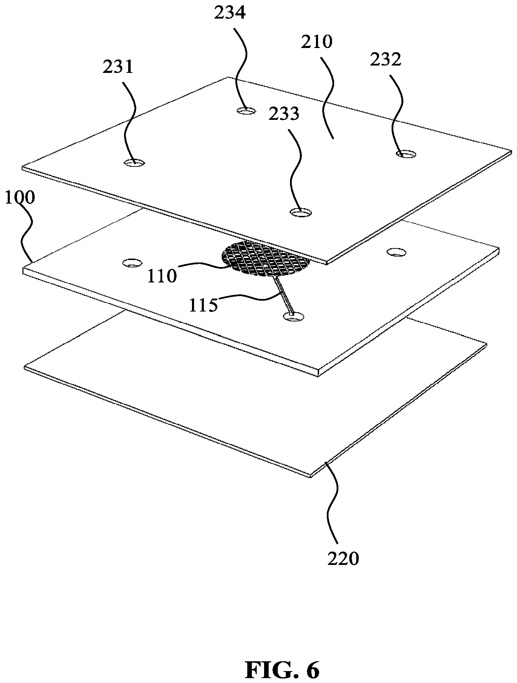

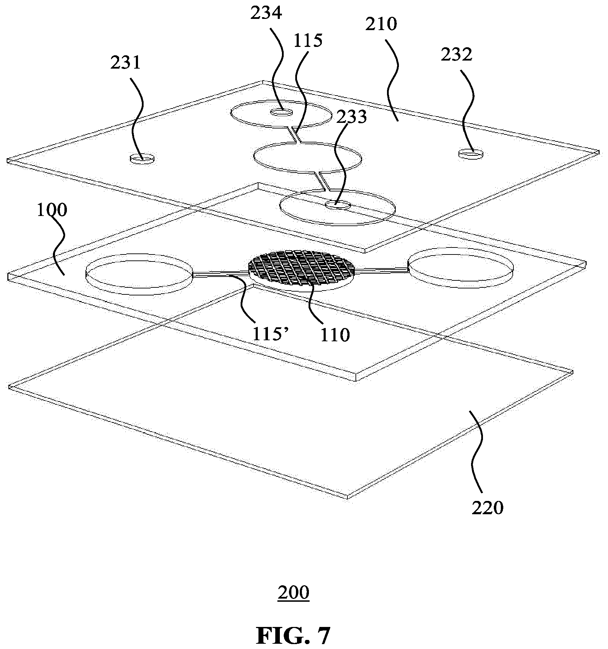

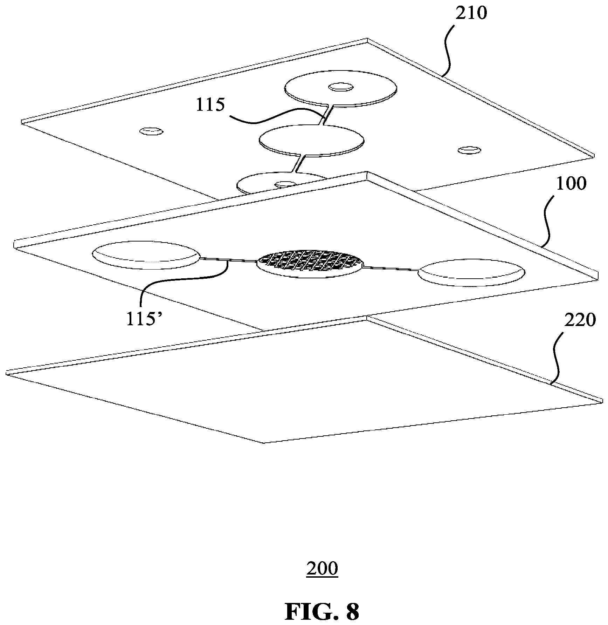

In an embodiment schematically depicted in FIG. 6, the upper and lower channels 115 are incorporated in the (monolithic) fluidic device module 100 (only one of the channels is shown), e.g. when molding the fluidic device module 100. Alternatively, at least one of the upper and lower channels may be formed in one of the cover plates. FIG. 7 schematically depicts a perspective top view and FIG. 8 schematically depicts a perspective bottom view of an example embodiment of a fluidic device 200 in which the upper channel 115 is formed in the upper cover plate 210 whilst the lower channel 115' is formed in the fluidic device module 100. It is of course equally feasible to form the lower channel 115' in the bottom cover plate 220 as will be readily understood by the skilled person. The fluidic device 200 such as the device shown in FIG. 7 and FIG. 8 for example may be used as a microwell in a lab on chip device in which a plurality of such microwells may be clamped or otherwise secured to facilitate parallel testing of a plurality of different samples within a single lab on chip device, in which each microwell typically comprises one of these samples.

Upon immobilizing a cell culture 50 in such a fluidic device 200 and proliferating this cell culture, the fluidic device 200 may be used in methods in which the immobilized proliferated cell culture 50 is exposed to a fluid comprising a compound of interest, which fluid may be passed through a flow channel 115 of the fluidic device 200 in order to expose the immobilized cell culture 50 to the compound of interest in the fluid and monitor the reaction of the immobilized cell culture 50 to such exposure. Such a method for example may be deployed in an oncology setting where the immobilized cell culture 50 may be exposed to a drug, e.g. a chemotherapy drug, in order to monitor the response of the immobilized cell culture 50 to such a drug. To this end, the immobilized cell culture 50 may include tumor cells in order to test the efficacy of the drug and/or may include healthy cells in order to test the toxicity of the drug on healthy tissue.

Such a drug testing method typically involves providing a fluidic device module 100 in accordance with one or more embodiments of the present invention, e.g. providing a monolithic fluidic device module 100 and applying a harvested cell culture to at least the membrane 110 where the cell culture can directly bind to the carboxylic acid or carboxylate groups on this exposed surface to obtain a prepared fluidic device module 100 from which a fluidic device 200, e.g. an organ on chip, is formed as previously explained. The cell culture within the fluid device 200 may be fed through one of a pair of flow channels 115 of the fluidic device 200 in order to keep the cell culture alive, whilst the cell culture may be exposed to a drug to be tested through the other of the pair of flow channels 115 of the fluidic device 200, with the drug testing method typically being completed by monitoring the response of the cell culture to the drug to be tested. This for example may be achieved by disassembling the fluidic device 200 by removing the cover plates 210, 220 and slicing the fluid device module 100 such that a slice of this module including a portion of the cell culture is obtained, which slice may be investigated under a microscope or the like in order to investigate the effects of the drug under test on the cell culture. During such investigations, the cell culture is typically stained, treated with formalin and fixated in a fixating agent such as paraffin to facilitate such microscopic investigation. Such staining, treating and fixating of the cell culture may be deployed within the fluidic device 200, e.g. by passing the appropriate chemical agents through a fluidic channel 115 of the fluidic device 200 in which the cell culture is exposed.

In an alternative embodiment, such disassembly of the fluidic device 200 can be avoided and the monitoring of the response of the cell culture to one or more compounds such as a drug to be tested can be achieved in real time using confocal microscopy. In particular, where embodiments of the fluidic device 200 comprise transparent cover plates 210, 220, e.g. glass plates or polymer plates, confocal microscopy may be used to monitor the cell culture within an assembled fluidic device 200. This is made possible through the fact that at least the bottom cover plate 220 can be kept thin, for example having a thickness in a range of 150-300 µm, with the distance between the membrane 110 of the fluidic device module 100 carrying the cell culture and the bottom cover plate 220 being less than 200 µm such that the focal length limits of confocal microscopy (typically around 500 µm from the object to be investigated to the lens objective). To this end, the membrane 110 may have a thickness in a range of 10-100 µm, such as a thickness of 20-30 µm. The thickness of the upper cover plate 210 is not critical; any suitable thickness may be contemplated, such as a thickness of 300 µm - 3 mm.

The evaluation of the cell culture within the fluidic device 200 using confocal microscopy is particularly suitable where the cell spheroids have a diameter of less than 500 µm, e.g. about 200 µm in order to ensure that the overall focal length of the optical path facilitates the capture of a sharp image with the confocal microscope. For larger spheroids, the fluidic device 200 may have to be dismantled such that the membrane 110 including the spheroids can be pressed onto a glass cover slip for positioning in the optical objective of the confocal microscope.

It is furthermore noted that the aforementioned dimensions of the fluidic device 200 typically apply during the real-time monitoring of the cell culture within the fluidic device 200. In embodiments in which the fluidic device 200 is sliced as previously explained, e.g. for digital pathology, the dimensions of the fluidic device 200 are less critical as long as the formalin and paraffin fixation can be performed thereon.

Above disclosed cell culturing substrates are just examples. Other such substrates exist. For one, such substrates may be a well cell plate. The substrate may be formed to be used with a well cell plate for example slabs of the substrate may be arranged to have shapes and sizes adapted such that they can be inserted into the standard sizes of wells of well cell plates. Alternatively, well cell plates with open wells may be used with portions of the material closing off the bottoms of the well cell plates. Such ensembles or arrangements may be clamped.

As will be readily understood by the skilled person, such drug testing methods may significantly differ in terms of protocol, e.g. drug dosages, administration frequencies, and so on. It should be understood that the drug testing method of the present invention is not limited to a particular protocol as long as such methods may be deployed using a fluidic device 200 according to an embodiment of the present invention in which cells are cultured directly onto an exposed surface, e.g. the membrane 110, of the device.

At this point, proof of concept of the cultivation of mammalian cells directly onto the bulk-modified elastomer of the present invention will be provided in the form of the following experimental evidence.

### General procedure:

Samples were cleaned by washing the samples in Iso-propanol for 5 minutes. After drying, the samples were placed in a 24 well plate. A cell culture of immortalized human prostate fibroblasts (WPMY-1 cells, ATCC CRL-2854) was provided in DMEM (Dulbecco's Modified Eagle Medium, Thermofisher) with 10% FBS (Feutal Bovine Serum), 1% Penicillin/Streptomycin and 1% Glutamax. A total amount of 2.0 × 10⁵ cells were seeded on all samples, and placed in an incubator overnight at 37°C and under an atmosphere of 5% CO₂. Images of the thus generated cell cultures were made using a Leica Inversed bright-field microscope at 100 times magnification.

Example 1: The WPMY-1 cell culture was developed in accordance with the general procedure as described above on a silicone elastomer bulk modified in accordance with the teachings of the present invention with 0.5 wt% linoleic acid. To this end, synthesis example 2 was amended by replacing the 4.848 g of sodium linoleate with 2.41 g of linoleic acid to obtain the bulk modified silicone elastomer used in this example.

Comparative Example 1: The WPMY-1 cell culture was developed in accordance with the general procedure as described above on the same silicone elastomer as example 1 but without bulk modification.

Example 2: The WPMY-1 cell culture was developed in accordance with the general procedure as described above on the bulk modified silicone elastomer prepared in accordance with synthesis example 2 (i.e. containing 1.0% by weight of sodium linoleate).

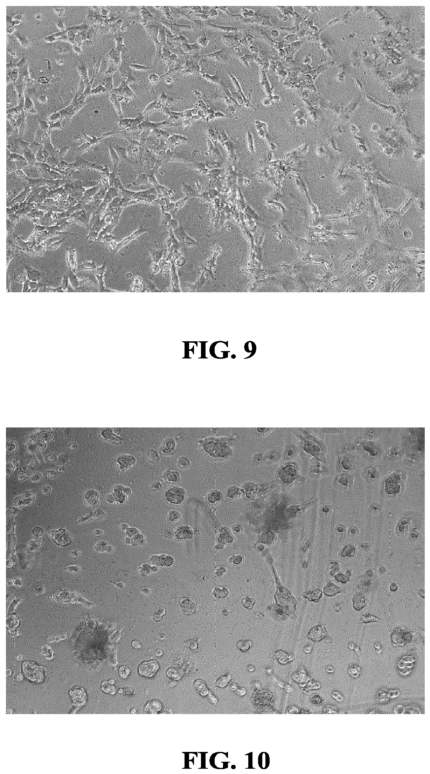

FIG. 9 shows the resulting cell culture of example 1 after the overnight cultivation. It can be clearly seen in this image that the cells show good adherence to the surface of the linoleic acid-functionalized silicone.

FIG. 10 shows the resulting cell culture of comparative example 1 after the overnight cultivation. It can be clearly seen in this image that the cells show minimal adherence to the surface of the unfunctionalized silicone elastomer.

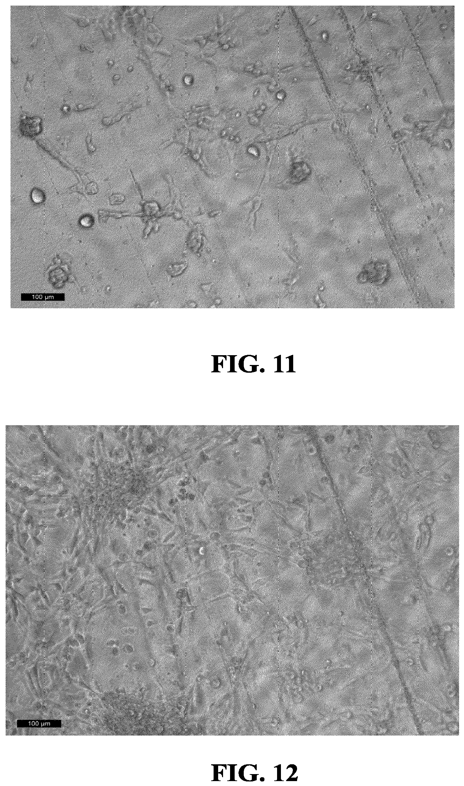

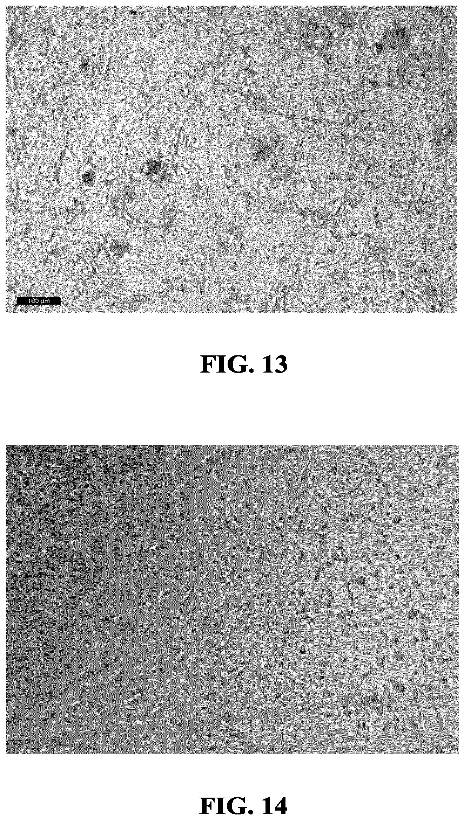

FIG. 11 shows the resulting cell culture of example 2 after the overnight cultivation. It can be clearly seen in this image that the cells show good adherence to the surface of the sodium linoleate-functionalized silicone. FIG. 12 shows the cell culture of example 2 after culturing for 2 days and FIG. 13 shows the cell culture of example 2 after culturing for 7 days. Ongoing cell proliferation can clearly be recognized from these images, which demonstrates that mammalian cell material can be directly cultured and proliferated onto the bulk-functionalized elastomers of the present invention.

Similar results were obtained using other cell types on substrates prepared and treated as for Example 2, but with a 1 wt% linoleic acid.

Example 3: The Endothelial cells Ea.hy926 cell culture was developed in accordance with the general procedure as described above on the bulk modified silicone elastomer prepared in accordance with synthesis example 2 (i.e. containing 1.0% by weight of sodium linoleate).

Example 4: The Epithelial cells Caco-2 cell culture was developed in accordance with the general procedure as described above on the bulk modified silicone elastomer prepared in accordance with synthesis example 2 (i.e. containing 1.0% by weight of sodium linoleate).

Figs 14 and 15 show the cell proliferation after 4 Days for example 3 and 4 respectively. Thus all cell lines show good proliferation after several days on the substrates. reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention can be implemented by means of hardware comprising several distinct elements. In the device claim enumerating several means, several of these means can be embodied by one and the same item of hardware. The mere fact that certain measures are recited in mutually different dependent claims does not indicate that a combination of these measures cannot be used to advantage.

## Claims

1. A cell culturing substrate comprising a material having an external surface for culturing cells thereon, the material containing a bulk-modified elastomer having an elastomer bulk and a plurality of residues each comprising one or more acidic groups in free and/or conjugated base form, the plurality of residues being covalently bound to the elastomer bulk as a result of a reaction between an unsaturated carbon-carbon bond of a precursor of the residue and a vinyl or hydride functional group of the elastomer bulk, such that part of the one or more acidic groups is available on the external surface, wherein the residue concentration in the bulk modified elastomer is in a range of 2.10⁻⁴ to 1.10⁻² mol/kg by weight of the total weight of the elastomer.

2. A substrate as claimed in claim 1 wherein the one or more acidic groups are chosen from the group consisting of phosphorus based acidic groups, sulfur based acidic groups and carboxylic acid groups or a mixture of two or more of these.

3. A substrate as claimed in any of the previous claims wherein the residues comprise or consist of an aliphatic portion comprising 3 or more carbon atoms and less than 50 carbon atoms, the one or more acidic groups being covalently coupled to the aliphatic portion and the aliphatic portion being covalently coupled to the elastomer bulk.

4. A substrate as claimed in any of the previous claims wherein the residues are unsaturated fatty acid residues.

5. A substrate as claimed in claim 4 wherein the unsaturated fatty acid residues are one or more residues of fatty acids chosen from the group consisting of: myristoleic acid, palmitoleic acid, sapienic acid, oleic acid, elaidic acid, vaccenic acid, linoleic acid, linoeladic acid, α-linolenic acid, arachidonic acid, eicospaentaenoic acid, erucic acid and docosahexaenoic acid.

6. A substrate as claimed in any of the previous claims wherein the elastomer bulk comprises a silicone backbone.

7. A substrate as claimed in any of claims 1 to 5 wherein the elastomer bulk comprises a polybutadiene backbone.

8. A substrate as claimed in any of the previous claims wherein the bulk-modified elastomer is obtainable or obtained by mixing a vinyl-functionalized and/or a hydride-functionalized elastomer or at least one precursor thereof and the residue precursor and heating the mixture, preferably the elastomer being a silicone based elastomer.

9. The substrate of claim 8 wherein the heating is performed in a reaction vessel having a metal metal oxide inner surface.

10. A cell culturing substrate as claimed in any of the claims 1 to 9, the substrate comprising a fluidic device module including a flow channel extending over a membrane comprising the material.

11. The cell culturing substrate of claim 10, comprising a first major surface comprising a first recessed structure defining a first flow channel and a second major surface opposing the first major surface and comprising a second recessed structure defining a second flow channel; wherein the membrane separates the first flow channel from the second flow channel.

12. The cell culturing substrate of claim 10 or 11, wherein the fluidic device is a monolithic fluidic device.

13. Use of a material having an external surface for culturing cells directly thereon, the material containing a bulk-modified elastomer having an elastomer bulk and a plurality of residues each comprising one or more acidic groups in free and/or conjugated base form, the plurality of residues being covalently bound to the elastomer bulk as a result of a reaction between an unsaturated carbon-carbon bond of a precursor of the residue and a vinyl or hydride functional group of the elastomer bulk, such that part of the one or more acidic groups is available on the external surface, wherein the residue concentration in the bulk-modified elastomer is in a range of 2.10⁻⁴ to 1.10⁻² mol/kg by weight of the total weight of the elastomer, the use comprising contacting the external surface with cells to be cultured and a cell growth medium.

14. A method of culturing cells, comprising the steps of:
using a cell culturing substrate as claimed in any of the previous claims, optionally, the substrate being a molded substrate; and
culturing cells directly on said external surface.

15. The method of claim 14, wherein said cell culturing substrate is one as claimed in any of the claims 10 to 12.

16. A drug testing method comprising the method of claim 15 and the further step of:
exposing the cultured cells to a drug to be tested through the other of the pair of flow channels of the fluidic device; and
monitoring the response of the cultured cells to the drug to be tested.

17. A method of manufacturing a cell culturing substrate comprising:
heating in a mold a mixture of a vinyl-functionalized and/or a hydride-functionalized elastomer or at least one precursor thereof and a residue precursor as defined herein in a concentration in the range of 2.10⁻⁴ to 1.10⁻² mol/kg by weight of the total weight of the mixture, wherein the residue precursor comprises one or more acidic groups in free and/or conjugated base form and an unsaturated carbon-carbon bond.

18. A mixture of a vinyl-functionalized and/or a hydride-functionalized elastomer or at least one precursor thereof and a residue precursor as defined herein in a concentration in the range of 2.10⁻⁴ to 1.10⁻² mol/kg by weight of the total weight of the mixture, wherein the residue precursor comprises one or more acidic groups in free and/or conjugated base form and an unsaturated carbon-carbon bond.

## Patentansprüche

1. Zellkultivierungssubstrat, umfassend ein Material, das eine Außenoberfläche zum Kultivieren von Zellen darauf aufweist, wobei das Material ein massenmodifiziertes Elastomer enthält, das eine Elastomermasse und eine Vielzahl von Resten aufweist, die jeweils eine oder mehrere Säuregruppen in freier und/oder konjugierter Basenform umfassen, wobei die Vielzahl von Resten infolge einer Reaktion zwischen einer ungesättigten Kohlenstoff-Kohlenstoff-Bindung eines Vorläufers des Rests und einer Vinyl- oder Hydrid-Funktionsgruppe der Elastomermasse kovalent an die Elastomermasse gebunden ist, sodass ein Teil der einen oder der mehreren Säuregruppen auf der Außenoberfläche verfügbar ist, wobei die Restkonzentration in dem massenmodifizierten Elastomer in einem Bereich von 2.10⁻⁴ bis 1.10⁻² mol/kg nach Gewicht, bezogen auf das Gesamtgewicht des Elastomers, liegt.

2. Substrat nach Anspruch 1, wobei die eine oder die mehreren Säuregruppen ausgewählt sind aus der Gruppe, bestehend aus phosphorbasierten Säuregruppen, schwefelbasierten Säuregruppen und Carbonsäuregruppen oder einer Mischung aus zwei oder mehreren dieser.

3. Substrat nach einem der vorstehenden Ansprüche, wobei die Reste einen aliphatischen Abschnitt umfassen oder daraus bestehen, der 3 oder mehr Kohlenstoffatome und weniger als 50 Kohlenstoffatomen umfasst, wobei die eine oder die mehreren Säuregruppen kovalent an den aliphatischen Abschnitt gekoppelt sind und der aliphatische Abschnitt kovalent an die Elastomermasse gekoppelt ist.

4. Substrat nach einem der vorstehenden Ansprüche, wobei die Reste ungesättigte Fettsäurereste sind.

5. Substrat nach Anspruch 4, wobei die ungesättigten Fettsäurereste ein oder mehrere Reste von Fettsäuren sind, die ausgewählt sind aus der Gruppe, bestehend aus Myristoleinsäure, Palmitoleinsäure, Sapiensäure, Ölsäure, Elaidinsäure, Vaccensäure, Linolsäure, Linoeladicsäure, α-Linolensäure, Arachidonsäure, Eicospaentaensäure, Erucasäure und Docosahexaensäure.

6. Substrat nach einem der vorstehenden Ansprüche, wobei die Elastomermasse ein Silikonrückgrat umfasst.

7. Substrat nach einem der Ansprüche 1 bis 5, wobei die Elastomermasse ein Polybutadienrückgrat umfasst.

8. Substrat nach einem der vorstehenden Ansprüche, wobei das massenmodifizierte Elastomer erhältlich ist oder erhalten wird, indem ein vinylfunktionalisiertes und/oder ein hydridfunktionalisiertes Elastomer oder mindestens ein Vorläufer davon und der Restvorläufer gemischt werden und die Mischung erhitzt wird, wobei das Elastomer vorzugsweise ein silikonbasiertes Elastomer ist.

9. Substrat nach Anspruch 8, wobei das Erhitzen in einem Reaktionsgefäß durchgeführt wird, das eine Metalloxid-Innenoberfläche aufweist.

10. Zellkultivierungssubstrat nach einem der Ansprüche 1 bis 9, wobei das Substrat ein Fluidvorrichtungsmodul umfasst, das einen Strömungskanal einschließt, der sich über eine Membran erstreckt, die das Material umfasst.

11. Zellkultivierungssubstrat nach Anspruch 10, umfassend eine erste Hauptoberfläche, die eine erste vertiefte Struktur umfasst, die einen ersten Strömungskanal definiert, und eine zweite Hauptoberfläche, die der ersten Hauptoberfläche gegenüberliegt und eine zweite vertiefte Struktur umfasst, die einen zweiten Strömungskanal definiert; wobei die Membran den ersten Strömungskanal vom zweiten Strömungskanal trennt.

12. Zellkultivierungssubstrat nach Anspruch 10 oder 11, wobei die Fluidvorrichtung eine monolithische Fluidvorrichtung ist.

13. Verwendung eines Materials, das eine Außenoberfläche zum Kultivieren von Zellen direkt darauf aufweist, wobei das Material ein massenmodifiziertes Elastomer enthält, das eine Elastomermasse und eine Vielzahl von Resten aufweist, die jeweils eine oder mehrere Säuregruppen in freier und/oder konjugierter Basenform umfassen, wobei die Vielzahl von Resten infolge einer Reaktion zwischen einer ungesättigten Kohlenstoff-Kohlenstoff-Bindung eines Vorläufers des Rests und einer Vinyl- oder Hydrid-Funktionsgruppe der Elastomermasse kovalent an die Elastomermasse gebunden ist, sodass ein Teil der einen oder der mehreren Säuregruppen auf der Außenoberfläche verfügbar ist, wobei die Restkonzentration in dem massenmodifizierten Elastomer in einem Bereich von 2.10⁻⁴ bis 1.10⁻² mol/kg nach Gewicht, bezogen auf das Gesamtgewicht des Elastomers, liegt.

14. Verfahren zum Kultivieren von Zellen, umfassend die Schritte von:
Verwenden eines Zellkultivierungssubstrats nach einem der vorstehenden Ansprüche, optional, wobei das Substrat ein geformtes Substrat ist; und
Kultivieren von Zellen direkt auf der Außenoberfläche.

15. Verfahren nach Anspruch 14, wobei das Zellkultivierungssubstrat eines nach einem der Ansprüche 10 bis 12 ist.

16. Arzneimitteltestverfahren, umfassend das Verfahren nach Anspruch 15 und den weiteren Schritt von:
Aussetzen der kultivierten Zellen einem zu testenden Arzneimittel durch den anderen des Paares von Strömungskanälen der Fluidvorrichtung; und
Überwachen der Reaktion der kultivierten Zellen auf das zu testende Arzneimittel.

17. Verfahren zur Herstellung eines Zellkultivierungssubstrats, umfassend:
Erhitzen einer Mischung aus einem vinylfunktionalisierten und/oder einem hydridfunktionalisierten Elastomer oder mindestens einem Vorläufer davon und einem Restvorläufer, wie hierin definiert, in einer Konzentration im Bereich von 2.10⁻⁴ bis 1.10⁻² mol/kg nach Gewicht, bezogen auf das Gesamtgewicht der Mischung, in einer Form, wobei der Restvorläufer eine oder mehrere Säuregruppen in freier und/oder konjugierter Basenform und eine ungesättigte Kohlenstoff-Kohlenstoff-Bindung umfasst.

18. Mischung aus einem vinylfunktionalisierten und/oder einem hydridfunktionalisierten Elastomer oder mindestens einem Vorläufer davon und einem Restvorläufer, wie hierin definiert, in einer Konzentration im Bereich von 2.10⁻⁴ bis 1.10⁻² mol/kg nach Gewicht, bezogen auf das Gesamtgewicht der Mischung, wobei der Restvorläufer eine oder mehrere Säuregruppen in freier und/oder konjugierter Basenform und eine ungesättigte Kohlenstoff-Kohlenstoff-Bindung umfasst.

## Revendications

1. Substrat de culture de cellules comprenant un matériau présentant une surface externe pour la culture de cellules sur celle-ci, le matériau contenant un élastomère modifié en masse présentant une masse d'élastomère et une pluralité de résidus comprenant chacun un ou plusieurs groupes acides sous forme de base libre et/ou conjuguée, la pluralité de résidus étant liés de manière covalente à la masse d'élastomère à la suite d'une réaction entre une liaison carbone-carbone insaturée d'un précurseur du résidu et un groupe fonctionnel vinyle ou hydrure de la masse d'élastomère, de sorte qu'une partie des un ou plusieurs groupes acides soit disponible sur la surface externe, dans lequel la concentration de résidus dans l'élastomère modifié en masse se situe dans une plage de 2,10⁻⁴ à 1,10⁻² mol/kg en poids du poids total de l'élastomère.

2. Substrat selon la revendication 1, dans lequel les un ou plusieurs groupes acides sont choisis dans le groupe consistant en des groupes acides à base de phosphore, des groupes acides à base de soufre et des groupes acides carboxyliques ou un mélange de deux ou de plusieurs d'entre eux.

3. Substrat selon l'une quelconque des revendications précédentes, dans lequel les résidus comprennent ou consistent en une partie aliphatique comprenant 3 atomes de carbone ou plus et moins de 50 atomes de carbone, les un ou plusieurs groupes acides étant couplés de manière covalente à la partie aliphatique et la partie aliphatique étant couplée de manière covalente à la masse d'élastomère.

4. Substrat selon l'une quelconque des revendications précédentes, dans lequel les résidus sont des résidus d'acides gras insaturés.

5. Substrat selon la revendication 4, dans lequel les résidus d'acides gras insaturés sont un ou plusieurs résidus d'acides gras choisis dans le groupe consistant en : l'acide myristoléique, l'acide palmitoléique, l'acide sapénique, l'acide oléique, l'acide élaïdique, l'acide vaccénique, l'acide linoléique, l'acide linoéladique, l'acide α-linolénique, l'acide arachidonique, l'acide eicospaentaénoïque, l'acide érucique et l'acide docosahexaénoïque.

6. Substrat selon l'une quelconque des revendications précédentes, dans lequel la masse d'élastomère comprend un squelette de silicone.

7. Substrat selon l'une quelconque des revendications 1 à 5, dans lequel la masse d'élastomère comprend un squelette de polybutadiène.

8. Substrat selon l'une quelconque des revendications précédentes, dans lequel l'élastomère modifié en masse peut être obtenu ou est obtenu en mélangeant un élastomère fonctionnalisé au vinyle et/ou fonctionnalisé à l'hydrure ou au moins un de ses précurseurs et le précurseur de résidus et en chauffant le mélange, l'élastomère étant de préférence un élastomère à base de silicone.

9. Substrat selon la revendication 8, dans lequel le chauffage est effectué dans une cuve de réaction présentant une surface intérieure en oxyde métallique.

10. Substrat de culture de cellules selon l'une quelconque des revendications 1 à 9, le substrat comprenant un module de dispositif fluidique incluant un canal d'écoulement s'étendant sur une membrane comprenant le matériau.

11. Substrat de culture de cellules selon la revendication 10, comprenant une première surface principale comprenant une première structure en creux définissant un premier canal d'écoulement et une seconde surface principale opposée à la première surface principale et comprenant une seconde structure en creux définissant un second canal d'écoulement ; dans lequel la membrane sépare le premier canal d'écoulement du second canal d'écoulement.

12. Substrat de culture de cellules selon la revendication 10 ou 11, dans lequel le dispositif fluidique est un dispositif fluidique monolithique.

13. Utilisation d'un matériau présentant une surface externe pour la culture de cellules directement sur celle-ci, le matériau contenant un élastomère modifié en masse présentant une masse d'élastomère et une pluralité de résidus comprenant chacun un ou plusieurs groupes acides sous forme de base libre et/ou conjuguée, la pluralité de résidus étant liés de manière covalente à la masse d'élastomère à la suite d'une réaction entre une liaison carbone-carbone insaturée d'un précurseur du résidu et un groupe fonctionnel vinyle ou hydrure de la masse d'élastomère, de sorte qu'une partie des un ou plusieurs groupes acides soit disponible sur la surface externe, dans lequel la concentration de résidus dans l'élastomère modifié en masse se situe dans une plage de 2,10⁻⁴ à 1,10⁻² mol/kg en poids du poids total de l'élastomère, l'utilisation comprenant la mise en contact de la surface externe avec des cellules à cultiver et un milieu de croissance cellulaire.

14. Procédé de culture de cellules, comprenant les étapes consistant à :
utiliser un substrat de culture de cellules tel que revendiqué dans l'une quelconque des revendications précédentes, le substrat étant facultativement un substrat moulé ; et
cultiver des cellules directement sur ladite surface externe.

15. Procédé selon la revendication 14, dans lequel ledit substrat de culture de cellules est un substrat tel que revendiqué dans l'une quelconque des revendications 10 à 12.

16. Procédé de test de médicament comprenant le procédé selon la revendication 15 et les étapes supplémentaires consistant à :
exposer les cellules cultivées à un médicament à tester par l'autre de la paire de canaux d'écoulement du dispositif fluidique ; et
surveiller la réaction des cellules cultivées au médicament à tester.

17. Procédé de fabrication d'un substrat de culture de cellules comprenant :
le chauffage dans un moule d'un mélange d'un élastomère fonctionnalisé au vinyle et/ou fonctionnalisé à l'hydrure ou d'au moins un de ses précurseurs et d'un précurseur de résidus tel que défini dans le présent document à une concentration dans la plage de 2,10⁻⁴ à 1,10⁻² mol/kg en poids du poids total du mélange, dans lequel le précurseur de résidus comprend un ou plusieurs groupes acides sous forme de base libre et/ou conjuguée et une liaison carbone-carbone insaturée.

18. Mélange d'un élastomère fonctionnalisé au vinyle et/ou fonctionnalisé à l'hydrure ou d'au moins un de ses précurseurs et d'un précurseur de résidus tel que défini dans le présent document à une concentration dans la plage de 2,10⁻⁴ à 1,10⁻² mol/kg en poids du poids total du mélange, dans lequel le précurseur de résidus comprend un ou plusieurs groupes acides sous forme de base libre et/ou conjuguée et une liaison carbone-carbone insaturée.
